# EUROPEAN PATENT APPLICATION

(11) **EP 4 825 856 A1**
(43) Date of publication of application: **21.10.2026**
(21) Application number: 24903489.3
(22) Date of filing: 26.11.2024
(51) Int. Cl.: C07D 417/12, A01N 43/78, A01P 3/00

(54) **N-SUBSTITUTED OXY-2-AMINOTHIAZOLECARBOXAMIDE COMPOUND OR SALT THEREOF AND AGRICULTURAL AND HORTICULTURAL BACTERICIDE**

(30) Priority: 15.12.2023 JP 2023212115
(71) Applicant: Ishihara Sangyo Kaisha, Ltd., Osaka-shi, Osaka 550-0002 (JP)
(72) Inventor: IWAMOTO Takuya, Osaka-shi, Osaka 550-0002 (JP); HAYASHI Hiroyuki, Osaka-shi, Osaka 550-0002 (JP); TANAKA Hisaki, Osaka-shi, Osaka 550-0002 (JP); OHNO Masanari, Osaka-shi, Osaka 550-0002 (JP); SENUMA Wakana, Osaka-shi, Osaka 550-0002 (JP)
(74) Representative: Wächtershäuser & Hartz Patentanwaltspartnerschaft mbB
(86) International application number: PCT/JP2024/041728
(87) International publication number: WO 2025/126830

(57) **Abstract**

To provide a novel compound having excellent controlling effects against harmful plant diseases.

A N-substituted oxy-2-aminothiazolecarboxamide compound represented by the formula (I) or a salt thereof: 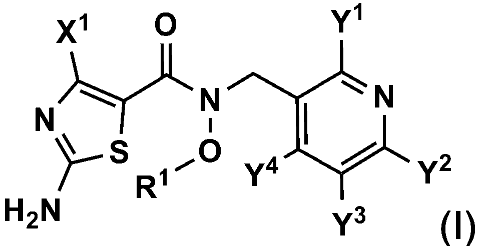 wherein the reference symbols are as defined in the specification, has excellent controlling effects against harmful plant diseases.

## Description

### TECHNICAL FIELD

The present invention relates to a novel N-substituted oxy-2-aminothiazolecarboxamide compound or a salt thereof, and an agricultural and horticultural fungicide comprising it as an active ingredient.

### BACKGROUND ART

Patent Document 1 discloses N-cycloalkyl-carboxamide, N-cycloalkyl-thiocarboxamide and N-cycloalkyl-N-substituted carboximidamide derivatives, and a method for controlling phytopathogenic fungi of crops, by using such compounds or compositions. However, Patent Document 1 does not at all disclose a N-substituted oxy-2-aminothiazolecarboxamide compound of the formula (I) described later.

### PRIOR ART DOCUMENTS

### PATENT DOCUMENTS

Patent Document 1: WO2008/037789

### DISCLOSURE OF INVENTION

### TECHNICAL PROBLEM

Existing agricultural and horticultural fungicides may be insufficient in practical controlling effects against harmful plant diseases in some application site, and may have problems of emergence of chemical resistant strains and of environmental burden (influence over surrounding plants and ecosystem). Accordingly, novel agricultural and horticultural fungicides have been desired. It is an object of the present invention to provide novel compounds having excellent controlling effects against harmful plant diseases.

### SOLUTION TO PROBLEM

The present inventors have conducted extensive studies to achieve the above object and as a result found that a N-substituted oxy-2-aminothiazolecarboxamide compound of the following formula (I) or a salt thereof, having a specific pyridin-3-ylmethyl group in its structure, has excellent controlling effects against harmful plant diseases problematic in agricultural and horticultural fields.

That is, the present invention provides the following.
[1] A N-substituted oxy-2-aminothiazolecarboxamide compound represented by the formula (I) or a salt thereof (hereinafter sometimes referred to as compound (I)): 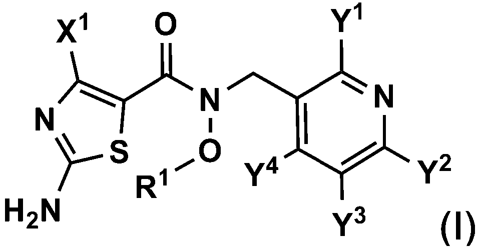
   wherein R¹ is a (C₁-C₆) chain hydrocarbon which may be substituted with at least one T¹,
   T¹ is cyano or -C(=O)O-R²,
   R² is a (C₁-C₃) alkyl,
   X' is a halogen, a (C₁-C₆) alkyl, a (C₁-C₆) haloalkyl, cyano or a hydrogen atom, and
   Y¹, Y², Y³ and Y⁴ are each independently a halogen, a hydrogen atom, a (C₁-C₆) alkyl, a (C₂-C₆) alkenyl, a (C₁-C₆) haloalkyl, cyano or nitro.
[2] The N-substituted oxy-2-aminothiazolecarboxamide compound or a salt thereof according to [1], wherein R¹ is a (C₁-C₆) alkyl or a (C₂-C₆) alkynyl.
[3] The N-substituted oxy-2-aminothiazolecarboxamide compound or a salt thereof according to [1], wherein R¹ is methyl, ethyl or propargyl.
[4] The N-substituted oxy-2-aminothiazolecarboxamide compound or a salt thereof according to any one of [1] to [3],
   wherein Y¹ and Y⁴ are each independently a halogen, a hydrogen atom, a (C₁-C₆) alkyl, a (C₁-C₆) haloalkyl, cyano or nitro, provided that Y¹ and Y⁴ are not hydrogen atoms at the same time, and
   Y² and Y³ are each independently a halogen or a hydrogen atom.
[5] The N-substituted oxy-2-aminothiazolecarboxamide compound or a salt thereof according to [1], wherein
   R¹ is a (C₁-C₆) chain hydrocarbon,
   X¹ is a halogen, a (C₁-C₆) alkyl, a (C₁-C₆) haloalkyl or a hydrogen atom, and
   Y¹, Y², Y³ and Y⁴ are each independently a halogen, a hydrogen atom, a (C₁-C₆) alkyl, a (C₁-C₆) haloalkyl or nitro.
[6] The N-substituted oxy-2-aminothiazolecarboxamide compound or a salt thereof according to [5], wherein R¹ is a (C₁-C₆) alkyl or a (C₂-C₆) alkynyl.
[7] The N-substituted oxy-2-aminothiazolecarboxamide compound or a salt thereof according to any one of [1], [4] and [5], wherein R¹ is methyl or propargyl.
[8] The N-substituted oxy-2-aminothiazolecarboxamide compound or a salt thereof according to any one of [1] to [3] and [5] to [7],
   wherein Y¹ is a halogen, a (C₁-C₆) alkyl, a (C₁-C₆) haloalkyl or nitro, and
   Y², Y³ and Y⁴ are each independently a halogen or a hydrogen atom.
[9] An agricultural and horticultural fungicide, which comprises as an active ingredient the N-substituted oxy-2-aminothiazolecarboxamide compound or a salt thereof as defined in any one of [1] to [8].
[10] A method for controlling harmful plant diseases, which comprises applying an effective amount of the N-substituted oxy-2-aminothiazolecarboxamide compound or a salt thereof as defined in any one of [1] to [8] to a plant body, phytopathogen or soil.

### ADVANTAGEOUS EFFECTS OF INVENTION

The compound (I) of the present invention has excellent controlling effects against harmful plant diseases and is useful as an agricultural and horticultural fungicide.

### DESCRIPTION OF EMBODIMENTS

Substituents and the chemical structure of the compound (I) will be described. In this specification, compounds included in the compound (I) will sometimes generically be referred to as Invention Compound.

In the compound (I), the halogen or the halogen as a substituent may, for example, be a fluorine, chlorine, bromine or iodine atom. The number of the halogen as the substituent may be one or more, and when two or more, the respective halogen atoms may be identical or different from one another. The position of the halogen as the substituent may be any position.

In the compound (I), the expression "C_{P}-C_{T}" means that the number of carbon atoms is P to T. For example, the expression "C₁-C₆" means that the number of carbon atoms is 1 to 6. Further, the expression "which may be substituted" means having a substituent or being unsubstituted.

The (C₁-C₆)-chain hydrocarbon means a (C₁-C₆)-alkyl, a (C₂-C₆)-alkynyl or a (C₂-C₆)-alkenyl.

The (C₁-C₆)-alkyl is a linear or branched alkyl group having 1 to 6 carbon atoms. It may, for example, be methyl, ethyl, n-propyl, isopropyl, n-butyl, isobutyl, s-butyl, tert-butyl, n-pentyl, isopentyl, neopentyl, s-pentyl, tert-pentyl, 2-methylbutyl, 3-pentyl, n-hexyl, isohexyl, s-hexyl, 2-methylpentyl, 3-methylpentyl, 3-hexyl, 2-ethylbutyl, 3-methylpentan-2-yl, 4-methylpentan-2-yl, 2,3-dimethylbutyl, tert-hexyl, 2,2-dimethylbutyl, neohexyl, 3-methylpentan-3-yl, 2-methylpentan-3-yl or 2,3-dimethylbutan-2-yl.

In this specification, the (C₁-C₃)-alkyl is a linear or branched alkyl group having 1 to 3 carbon atoms. Its specific examples correspond to the alkyl groups having 1 to 3 carbon atoms among the above specific examples of the (C₁-C₆)-alkyl.

The (C₂-C₆)-alkynyl is a linear or branched alkynyl group having 2 to 6 carbon atoms, having at least one triple bond at an optional position. It may, for example, be ethynyl, 1-propynyl, propargyl (hereinafter sometimes referred to simply as 2-propynyl), 1-butynyl, 2-butynyl, 3-butynyl, 3-butyn-2-yl, 1,3-butadiynyl, 1-pentynyl, 2-pentynyl, 3-pentynyl, 4-pentynyl, 3-methyl-1-butynyl, 3-pentyn-2-yl, 1-pentyn-3-yl, 4-pentyn-2-yl, 2-methyl-3-butynyl, 1,3-pentadiynyl, 1,4-pentadiynyl, 2,4-pentadiynyl, 1-hexynyl, 2-hexynyl, 3-hexynyl, 4-hexynyl, 5-hexynyl, 4-methyl-1-pentynyl, 3-methyl-1-pentynyl, 3,3-dimethyl-1-butynyl, 4-methyl-2-pentynyl, 3-hexyn-2-yl, 4-hexyn-2-yl, 2-methyl-3-pentynyl, 3-methyl-4-pentyn-2-yl, 5-hexyn-2-yl, 2,4-hexadiynyl, 3,5-hexadiynyl, 3,5-hexadiyn-2-yl or 1,3,5-hexatriynyl.

In this specification, the (C₂-C₃)-alkynyl is a linear alkynyl group having 2 to 3 carbon atoms, having at least one triple bond at an optional position. Its specific examples correspond to the alkynyl groups having 2 to 3 carbon atoms among the above specific examples of the (C₂-C₆) alkynyl.

The (C₂-C₆)-alkenyl is a linear or branched alkenyl group having 2 to 6 carbon atoms, having at least one double bond at an optional position. It may, for example, be vinyl (hereinafter sometimes referred to simply as ethenyl), allyl (hereinafter sometimes referred to simply as 2-propenyl), 1-propenyl, isopropenyl, 1-butenyl, 2-butenyl, 3-butenyl, 2-buten-2-yl, 3-buten-2-yl, 2-methyl-1-propenyl, 2-methyl-2-propenyl, 3-buten-3-yl, 1,3-butadienyl, 1,3-butadien-2-yl, 1-pentenyl, 2-pentenyl, 3-pentenyl, 4-pentenyl, 3-methyl-1-butenyl, 2-methyl-1-butenyl, 2-penten-1-yl, 3-methyl-2-buten-2-yl, 3-methyl-2-butenyl, 2-methyl-2-butenyl, 3-penten-2-yl, 3-methyl-3-butenyl, 2-methyl-3-butenyl, 1,3-pentadienyl, 2,4-pentadienyl, 2,4-pentadien-2-yl, 1-hexenyl, 2-hexenyl, 3-hexenyl, 4-hexenyl, 5-hexenyl, 2-hexen-2-yl, 2-ethyl-1-butenyl, 4-methyl-3-pentenyl, 3-methyl-3-pentenyl, 2-methyl-3-pentenyl, 2,3-dimethyl-2-butenyl, 4-methyl-3-penten-2-yl, 4-methyl-3-pentenyl, 3-methyl-3-pentenyl, 1,3-hexadienyl, 1,4-hexadienyl, 1,5-hexadienyl, 2,4-hexadienyl, 2,5-hexadienyl or 1,3,5-hexatrienyl. Further, in a case where such a group has geometrical isomers, the group may be one of E-isomer and Z-isomer or may be a mixture of E-isomer and Z-isomer in an optional ratio and is not particularly limited so long as it has carbon atoms within a specified range.

The (C₁-C₆) chain hydrocarbon may be substituted with at least one T¹. In a case where the (C₁-C₆) chain hydrocarbon is substituted with T¹, the (C₁-C₆) chain hydrocarbon can be substituted with 1 to 13 T¹.

In a case where the (C₁-C₆) chain hydrocarbon is substituted with at least one T¹, the position of T¹ as the substituent may be any position on the (C₁-C₆) chain hydrocarbon.

In a case where the (C₁-C₆) chain hydrocarbon is substituted with two or more T¹, the respective substituents T¹ may be identical or different from one another.

For example, in a case where R¹ in the compound (I) is a (C₁-C₆)-alkyl substituted with at least one T¹, specifically, the following substituents are included. For example, cyanomethyl, 2-cyanoethyl, 3-cyanopropyl, 4-cyanobutyl, 5-cyanopentyl, 6-cyanohexyl, methoxycarbonylmethyl, ethoxycarbonylmethyl, propoxycarbonylmethyl, 2-(methoxycarbonyl)ethyl and isopropoxycarbonylmethyl may be mentioned.

The (C₁-C₆) haloalkyl is a linear or branched alkyl group having 1 to 6 carbon atoms, partially or completely substituted with 1 to 13 halogen atoms which are identical or different from one another. It may, for example, be fluoromethyl, difluoromethyl, trifluoromethyl, chloromethyl, dichloromethyl, trichloromethyl, bromomethyl, dibromomethyl, tribromomethyl, chlorodifluoromethyl, dichlorofluoromethyl, chlorofluoromethyl, 1-fluoroethyl, 2-fluoroethyl, 1,1-difluoroethyl, 2,2-difluoroethyl, 2,2,2-trifluoroethyl, perfluoroethyl, 1-chloroethyl, 2-chloroethyl, 1,1-dichloroethyl, 2,2-dichloroethyl, 2,2,2-trichloroethyl, 1-chloro-1-fluoroethyl, 2-chloro-2-fluoroethyl, 1-fluoropropyl, 2-fluoropropyl, 1-fluoro-2-propyl, 3-fluoropropyl, 2-fluoro-2-propyl, 1,1-difluoropropyl, 2,2-difluoropropyl, 3,3-difluoropropyl, 3,3,3-trifluoropropyl, perfluoropropyl, perfluoroisopropyl, 2,2,3,3,3-pentafluoropropyl, 1,1,1,3,3,3-hexafluoroisopropyl, 1-fluorobutyl, 2-fluorobutyl, 3-fluorobutyl, 4-fluorobutyl, 1,1-difluorobutyl, 2,2-difluorobutyl, 3,3-difluorobutyl, 4,4-difluorobutyl, 4,4,4-trifluorobutyl, 3,3,4,4,4-pentafluorobutyl, 2,2,3,3,4,4,4-heptafluorobutyl, perfluorobutyl, 2,3,3,3,4,4,4-heptafluoroisobutyl, 1-fluoropentyl, 2-fluoropentyl, 3-fluoropentyl, 4-fluoropentyl, 5-fluoropentyl, 1,1-difluoropentyl, 2,2-difluoropentyl, 3,3-difluoropentyl, 4,4-difluoropentyl, 5,5-difluoropentyl, 5,5,5-trifluoropentyl, 4,4,5,5,5-pentafluoropentyl, 3,3,4,4,5,5,5-heptafluoropentyl, 2,2,3,3,4,4,5,5,5-nonafluoropentyl, perfluoropentyl, 1-fluorohexyl, 2-fluorohexyl, 3-fluorohexyl, 4-fluorohexyl, 5-fluorohexyl, 6-fluorohexyl, 1,1-difluorohexyl, 2,2-difluorohexyl, 3,3-difluorohexyl, 4,4-difluorohexyl, 5,5-difluorohexyl, 6,6-difluorohexyl, 6,6,6-trifluorohexyl, 5,5,6,6,6-pentafluorohexyl, 4,4,5,5,6,6,6-heptafluorohexyl, 3,3,4,4,5,5,6,6,6-nonafluorohexyl, 2,2,3,3,4,4,5,5,6,6,6-undecafluorohexyl or perfluorohexyl.

In this specification, the (C₁-C₃) haloalkyl is a linear or branched alkyl group having 1 to 3 carbon atoms, partially or completely substituted with 1 to 7 halogen atoms which are identical or different from one another. Specific examples of the (C₁-C₃) haloalkyl correspond to the haloalkyl groups having 1 to 3 carbon atoms among the above specific examples of the (C₁-C₆) haloalkyl.

The salts of the compound (I) include all kinds of salts so long as they are agriculturally acceptable, and, for example, alkali metal salts (such as a sodium salt and a potassium salt), alkaline earth metal salts (such as a magnesium salt and a calcium salt), amine salts (such as a dimethylamine salt and a triethylamine salt), inorganic acid salts (such as a hydrochloride, a perchlorate, a sulfate and a nitrate), and organic acid salts (such as an acetate, a methanesulfonate, a p-toluenesulfonate and an oxalate) may be mentioned.

As the compound (I), isomers such as optical isomers and geometrical isomers may sometimes be present, and the present invention includes the respective isomers and isomer mixtures. In the compound (I), various isomers other than the above-mentioned isomers are included within a range of common knowledge in this technical field. Further, an individual isomer may be synthesized by common knowledge and general experimental means in this technical field.

Further, depending upon the type of an isomer, it may have a chemical structure different from the described structural formula, but for those skilled in the art, it is sufficiently recognizable that such a chemical structure is in a relation of an isomer, and such an isomer is evidently within the scope of the present invention.

Now, the method for producing the compound (I) will be described.

The compound (I) may be produced in accordance with the following Reactions A to E and a conventional method for producing a salt, however, the method to obtain the compound is not limited to such a method. For example, the compound (I) of the present invention may be produced by applying a substituent transformation (such as alkylation, haloalkylation, cross coupling reaction such as Suzuki coupling, Sandmeyer reaction, halogenation, oxidation or reduction) known in this technical field, on the substituent on the pyridine ring. Further, in the production of the Invention Compound, protection and deprotection reaction commonly employed in this technical field may be applied as the case requires. The reaction may be carried out under an atmosphere of an inert gas such as nitrogen or argon as the case requires, and a salt reagent may be used.

### [Reaction A]

Reaction A is a deprotection reaction, which is a method of removing a Boc group from a compound of the formula (XX-a) to obtain a compound of the formula (I). The Boc group is a tert-butoxycarbonyl group.

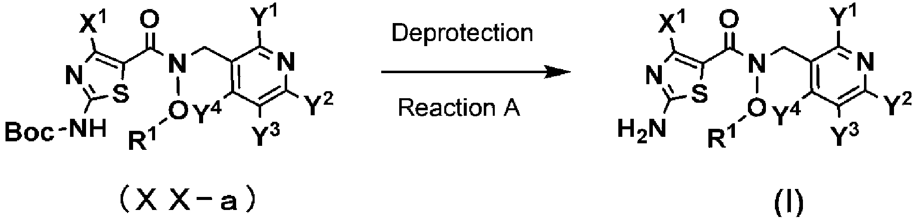

Symbols in the formulae are as defined above.

Reaction A may be carried out under known conditions employed to remove the Boc group, for example, in accordance with the method described in Greene's PROTECTIVE GROUPS in ORGANIC SYNTHESIS (John Wiley and Sons, 2007, Peter G.M.Wuts, Theodora W.Greene). More specifically, for example, the compound of the formula (XX-a) is reacted with an acid such as trifluoroacetic acid or hydrogen chloride in the presence of a solvent, or reacted with trimethylsilyl triflate in the presence of a solvent and a base such as 2,6-lutidine.

### [Reaction B] and [Reaction C]

Reaction B is a method of reacting a compound of the formula (II) with a compound of the formula (III) to obtain a compound of the formula (XX-b). Reaction C is a method of reacting a compound of the formula (II-a) with a compound of the formula (III) to obtain a compound of the formula (XX-b).

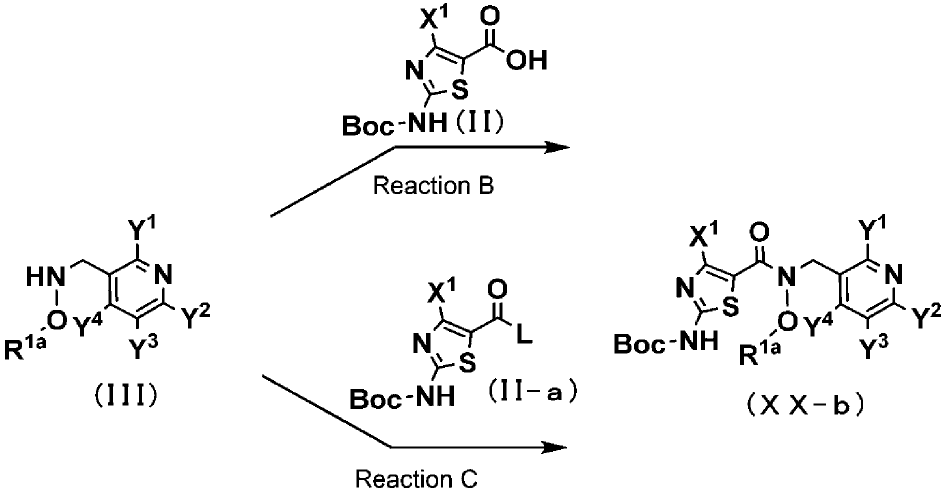

In the formulae in each Reaction, R^{1a} is H or a (C₁-C₆) chain hydrocarbon which may be substituted by at least one T¹, and L is a leaving group, such as a halogen, an alkoxy, an aryloxy, an alkylcarbonyloxy or an arylcarbonyloxy. The other symbols are as defined above.

Reaction B may be carried out usually in the presence of a dehydration condensation agent and a solvent, and optionally with the addition of a base. In Reaction B, the compound of the formula (III) may be used in an amount of 0.5 to 3 equivalents, preferably 0.8 to 1.5 equivalents to 1 equivalent of the compound of the formula (II) (equivalents means molar equivalents, the same applies hereinafter).

In Reaction B, the dehydration condensation agent may, for example, be a carbodiimide condensation agent such as N,N'-dicyclohexylcarbodiimide (DCC), 1-[3-(dimethylamino)propyl]-3-ethylcarbodiimide (EDC) or its hydrochloride; an imidazole condensation agent such as 1,1'-carbonyldiimidazole (CDI); a triazine condensation agent such as 4-(4,6-dimethoxy-1,3,5-triazin-2-yl)-4-methylmorphonium chloride (DMT-MM); a phosphonium condensation agent such as 1H-benzotriazol-1-yloxy-tripyrrolidinophosphonium hexafluorophosphate (PyBOP); an uronium condensation agent such as 1-[bis(dimethylamino)methylene]-1H-1,2,3-triazolo[4,5-b]pyridinium 3-oxide hexafluorophosphate (HATU); an acid anhydride such as 2-methyl-6-nitrobenzoic anhydride (MNBA); a 2-halopyridinium salt such as 2-chloro-1-methylpyridinium p-toluenesulfonate; propylphosphonic acid anhydride (cyclic trimer) (T3P); and diphenylphosphoryl azide (DPPA); but is not limited thereto. Optionally, a conventional additive used in combination with a dehydration condensation agent, such as 1-hydroxybenzotriazole (HOBt), may be added. The dehydration condensation agent may be used in an amount of 0.5 to 5 equivalents, preferably 1 to 2 equivalents to 1 equivalent of the compound of the formula (II), and the additive may be used in an amount of 0.2 to 5 equivalents, preferably 1 to 2 equivalents to 1 equivalent of the compound of the formula (II).

In Reaction B, as the base, one or more may properly be selected and mixed from carbonates such as sodium carbonate, potassium carbonate and cesium carbonate; hydrogencarbonates such as sodium hydrogencarbonate and potassium hydrogencarbonate; metal hydroxides such as sodium hydroxide and potassium hydroxide; metal hydrides such as sodium hydride and potassium hydride; amines such as triethylamine and N,N-diisopropylethylamine; pyridines such as pyridine, 4-dimethylaminopyridine and 2,6-lutidine; alkali metal carboxylates such as sodium acetate and potassium acetate; and the like. The base may be used in an amount of 0.5 to 10 equivalents, preferably 1 to 5 equivalents to 1 equivalent of the compound of the formula (II).

In Reaction B, the solvent may be any solvent so long as it is inert to the reaction, and one or more may properly be selected from aromatic hydrocarbons such as benzene, toluene, xylene and chlorobenzene; aliphatic hydrocarbons such as carbon tetrachloride, methyl chloride, chloroform, dichloromethane, dichloroethane, trichloroethane, hexane and cyclohexane; ethers such as dioxane, tetrahydrofuran, diethyl ether and dimethoxyethane; esters such as methyl acetate and ethyl acetate; aprotic polar solvents such as dimethyl sulfoxide, sulfolane, N,N-dimethylacetamide, N,N-dimethylformamide, N-methylpyrrolidone, pyridine, acetonitrile and propionitrile; ketones such as acetone and methyl ethyl ketone; protic polar solvents such as methanol and ethanol; water; and the like.

In Reaction B, the reaction temperature is usually about -20°C to 150°C, preferably about 0°C to 100°C, and the reaction time is usually about 0.5 to 48 hours, preferably about 1 to 24 hours.

Reaction C may be carried out in the presence of a solvent, and optionally with the addition of a base. In Reaction C, the compound of the formula (III) may be used in an amount of 0.5 to 3 equivalents, preferably 0.8 to 1.5 equivalents to 1 equivalent of the compound of the formula (II-a).

In Reaction C, as the base, one or more may properly be selected and mixed from carbonates such as sodium carbonate, potassium carbonate and cesium carbonate; hydrogencarbonates such as sodium hydrogencarbonate and potassium hydrogencarbonate; metal hydroxides such as sodium hydroxide and potassium hydroxide; metal hydrides such as sodium hydride and potassium hydride; amines such as triethylamine and N,N-diisopropylethylamine; pyridines such as pyridine, 4-dimethylaminopyridine and 2,6-lutidine; alkali metal carboxylates such as sodium acetate and potassium acetate; and the like. The base may be used in an amount of 0.1 to 10 equivalents, preferably 0.5 to 5 equivalents to 1 equivalent of the compound of the formula (II-a).

In Reaction C, the solvent may be any solvent so long as it is inert to the reaction, and one or more may properly be selected from aromatic hydrocarbons such as benzene, toluene, xylene and chlorobenzene; aliphatic hydrocarbons such as carbon tetrachloride, methyl chloride, chloroform, dichloromethane, dichloroethane, trichloroethane, hexane and cyclohexane; ethers such as dioxane, tetrahydrofuran, diethyl ether and dimethoxyethane; esters such as methyl acetate and ethyl acetate; aprotic polar solvents such as dimethyl sulfoxide, sulfolane, N,N-dimethylacetamide, N,N-dimethylformamide, N-methylpyrrolidone, pyridine, acetonitrile and propionitrile; ketones such as acetone and methyl ethyl ketone; protic polar solvents such as methanol and ethanol; water; and the like.

In Reaction C, the reaction temperature is usually about -20°C to 150°C, preferably about 0°C to 100°C, and the reaction time is usually about 0.5 to 48 hours, preferably about 1 to 24 hours.

The compound of the formula (III) used in Reaction B and Reaction C may be produced in accordance with the following Reaction 2-2 or Reaction 2-4.

The compound of the formula (II) used in Reaction B may be produced in accordance with the following Reaction 1-1, Reaction 1-2 or a known method, or may be commercially available.

The compound of the formula (II-a) used in Reaction C may be produced from the compound of the formula (II) in accordance with the following Reaction 1-4 or a known method, or may be commercially available.

### [Reaction D]

Reaction D is a method of reacting a compound of the formula (XX-c) with a compound of the formula (IV) to obtain the compound of the formula (XX-a).

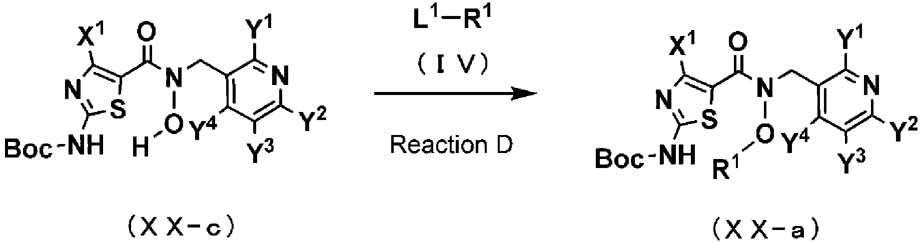

In the formulae, L¹ is a leaving group, such as a halogen, trifluoromethanesulfonyloxy, methanesulfonyloxy or p-toluenesulfonyloxy, and the other symbols are as defined above.

Reaction D may be carried out usually in the presence of a base and a solvent, and optionally with the addition of a phase transfer catalyst. In Reaction D, the compound of the formula (IV) may be used in an amount of 1 to 5 equivalents, preferably 1 to 3 equivalents to 1 equivalent of the compound of the formula (XX-c).

In Reaction D, as the base, one or more may properly be selected and mixed from alkali metal alkoxides such as sodium methoxide, sodium ethoxide and potassium tert-butoxide; carbonates such as sodium carbonate, potassium carbonate and cesium carbonate; hydrogencarbonates such as sodium hydrogencarbonate and potassium hydrogencarbonate; metal hydroxides such as sodium hydroxide and potassium hydroxide; metal hydrides such as sodium hydride and potassium hydride; amines such as triethylamine and N,N-diisopropylethylamine; pyridines such as pyridine, 4-dimethylaminopyridine and 2,6-lutidine; organic lithium compounds such as n-butyllithium and lithium diisopropylamide; alkali metal carboxylates such as sodium acetate and potassium acetate; and the like. The base may be used in an amount of 1 to 10 equivalents, preferably 1 to 5 equivalents to 1 equivalent of the compound of the formula (XX-c).

In Reaction D, the solvent may be any solvent so long as it is inert to the reaction, and one or more may properly be selected from aromatic hydrocarbons such as benzene, toluene, xylene and chlorobenzene; aliphatic hydrocarbons such as carbon tetrachloride, methyl chloride, chloroform, dichloromethane, dichloroethane, trichloroethane, hexane and cyclohexane; ethers such as dioxane, tetrahydrofuran, diethyl ether and dimethoxyethane; esters such as methyl acetate and ethyl acetate; alcohols such as methanol, ethanol, propanol and tert-butanol; aprotic polar solvents such as dimethyl sulfoxide, sulfolane, N,N-dimethylacetamide, N,N-dimethylformamide, N-methylpyrrolidone, pyridine, acetonitrile and propionitrile; ketones such as acetone and methyl ethyl ketone; water; and the like.

In Reaction D, the phase transfer catalyst may, for example, be quaternary ammonium salts such as tetrabutylammonium bromide, benzyltriethylammonium chloride or tetrabutylammonium hydrogen sulfate; or crown ethers such as 18-crown-6-ether. The phase transfer catalyst may be used in an amount of 0.1 to 3 equivalents to 1 equivalent of the compound of the formula (XX-c).

In Reaction D, the reaction temperature is usually about -20°C to 150°C, preferably about 0°C to 100°C, and the reaction time is usually about 10 minutes to 48 hours, preferably about 1 to 24 hours.

The compound of the formula (IV) used in Reaction D may be produced in accordance with a known method, or may be commercially available.

### [Reaction E]

Reaction E is a method of reacting a compound of the formula (II-b) with a compound of the formula (V) to obtain the compound of the formula (XX-a).

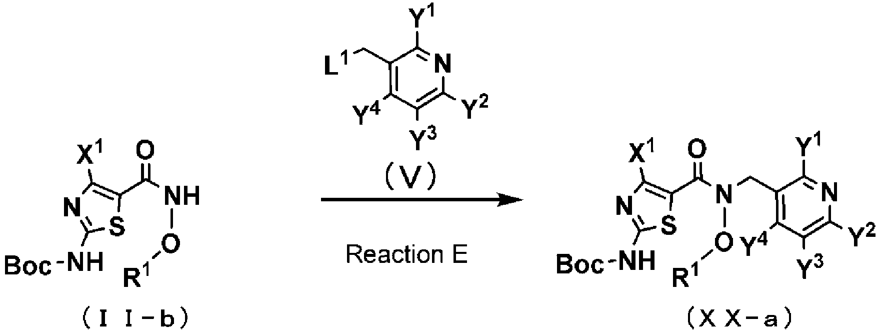

Symbols in the formulae are as defined above.

Reaction E may be carried out in accordance with the above Reaction D. In Reaction E, the compound of the formula (V) may be used in an amount of 1 to 5 equivalents, preferably 1 to 2 equivalents to 1 equivalent of the compound of the formula (II-b). In Reaction E, the base may be used in an amount of 1 to 10 equivalents, preferably about 1 to 5 equivalents to 1 equivalent of the compound of the formula (II-b). In Reaction E, the phase transfer catalyst may be used in an amount of 0.1 to 3 equivalents to 1 equivalent of the compound of the formula (II-b).

The compound of the formula (II-b) used in Reaction E may be produced in accordance with the following Reaction 1-3 or a known method, or may be commercially available. The compound of the formula (V) used in Reaction E may be produced in accordance with the following Reaction 2-7, 2-8 or 2-9, or a known method, or may be commercially available.

The compounds used in Reaction A to Reaction E may be produced in accordance with the following Intermediate Production Process (Reaction 1-1 to Reaction 1-5 and Reaction 2-1 to Reaction 2-9) and a conventional method for producing a salt, but their production is not limited to these methods. Such compounds may be produced in accordance with a known method, or may be commercially available.

### Intermediate Production Process

### [Reaction 1-1] to [Reaction 1-5]

Reaction 1-1 is a method of oxidizing a compound of the formula (1) to obtain the compound of the formula (II). Reaction 1-2 is a method of hydrolyzing a compound of the formula (2) to obtain the compound of the formula (II).

Reaction 1-3 is a method of reacting the compound of the formula (II) or the compound of the formula (II-a) with a compound of the formula (3) to obtain the compound of the formula (II-b).

Reaction 1-4 is a method of halogenating, esterifying or carbonylating the compound of the formula (II) to obtain the compound of the formula (II-a).

Reaction 1-5 is a method of protecting the amino group in a compound of the formula (VI) with a Boc group to obtain a compound of the formula (VII).

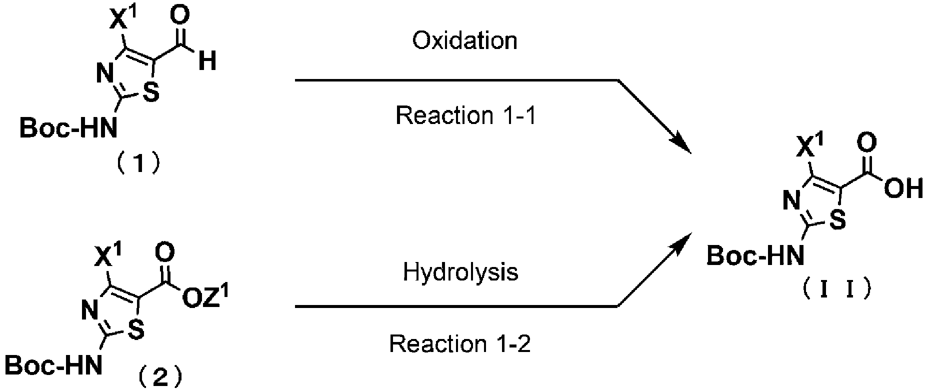

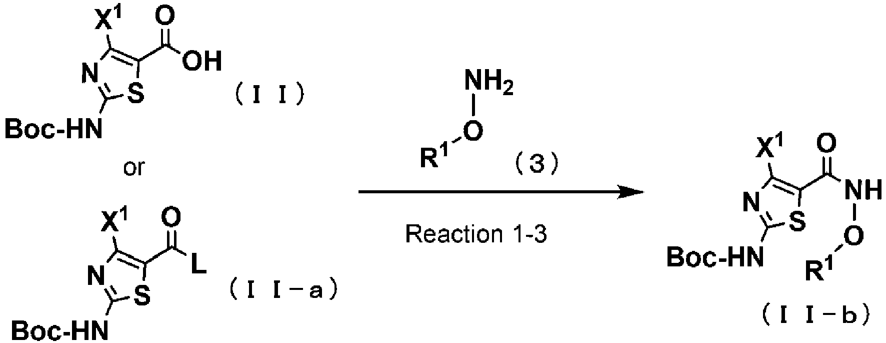

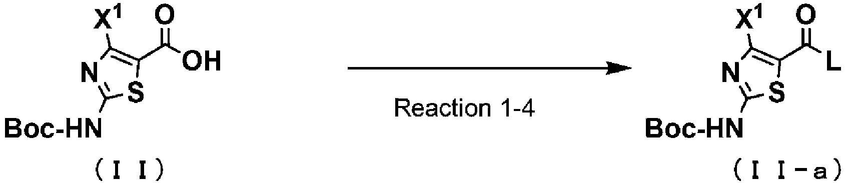

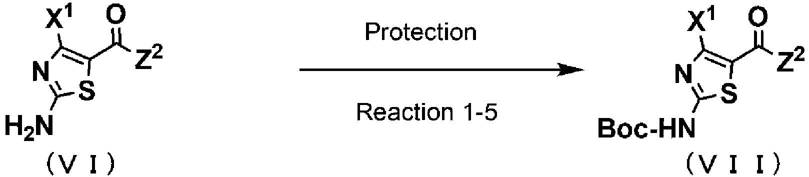

In the formulae in each Reaction, Z¹ is an alkyl, Z² is H or an alkyloxy, and the other symbols are as defined above.

Reaction 1-1 may be carried out under conventional Pinnick oxidation conditions, for example, in accordance with the method described in Bioorganic & Medicinal Chemistry, 2004, 12, 6171-6182.

The compound of the formula (1) used in Reaction 1-1 may be produced by a known method, for example, in accordance with the method described in Bioorganic & Medicinal Chemistry, 2004, 12, 6171-6182, Journal of Organic Chemistry, 2005, 70, 567-574, WO2020/028141, or Organic Process Research and Development, 2021, 25, 1167-1175 or Reaction 1-5, or may be commercially available.

Reaction 1-2 may be carried out under conventional conditions for hydrolysis of esters, for example, in accordance with the method described in WO2009/100171.

The compound of the formula (2) used in Reaction 1-2 may be produced by a known method, for example, in accordance with the method described in WO2012/006760, Japanese Patent No. 5851663 or Reaction 1-5, or may be commercially available.

Reaction 1-3 may be carried out in accordance with the above Reaction B or Reaction C. In Reaction 1-3, the compound of the formula (3) may be used in an amount of 0.5 to 10 equivalents, preferably 0.7 to 5 equivalents to 1 equivalent of the compound of the formula (II) or the compound of the formula (II-a).

The compound of the formula (3) used in Reaction 1-3 may be produced in accordance with a known method, for example, in accordance with the method described in WO2006/138350 or US Publication 2014/0378399, or may be commercially available.

The compound of the formula (II-a) used in Reaction 1-3 may be produced in accordance with a known method or the method described in Reaction 1-4, or may be commercially available.

Reaction 1-4, in a case where L in the compound of the formula (II-a) is a halogen, can be carried out usually by reacting the compound of the formula (II) with a halogenating agent in the presence of a solvent, and optionally with the addition of N,N-dimethylformamide.

In Reaction 1-4, the halogenating agent may, for example, be oxalyl chloride, thionyl chloride, phosphorus oxychloride, phosphorus oxybromide, phosphorus trichloride, phosphorus tribromide, phosphorus pentachloride or sulfuryl chloride. The halogenating agent may be used in an amount of 1 to 10 equivalents, preferably 1 to 3 equivalents to 1 equivalent of the compound of the formula (II), or may be used in excess within a range not to impair the reaction.

In Reaction 1-4, in a case where N,N-dimethylformamide is used, the amount of N,N-dimethylformamide is a catalytic amount and is for example 0.01 to 0.3 equivalents to 1 equivalent of the compound of the formula (II).

In Reaction 1-4, in a case where L in the compound of the formula (II-a) is an alkoxy or an aryloxy, the compound of the formula (II) is reacted with an alcohol or an arylhydroxy usually in the presence of a solvent and a dehydration condensation agent, and optionally with the addition of a base, for esterification.

In Reaction 1-4, the alcohol may, for example, be methanol or ethanol. In Reaction 1-4, the arylhydroxy may, for example, be phenol. The alcohol and the arylhydroxy may be used in an amount of 0.5 to 5 equivalents, preferably 0.8 to 1.5 equivalents, to 1 equivalent of the compound of the formula (II), or may be used in excess within a range not to impair the reaction.

In Reaction 1-4, as the dehydration condensation agent, ones mentioned for the above Reaction B may be used. In Reaction 1-4, in a case where the dehydration condensation agent is used, optionally a conventional additive (for example 1-hydroxybenzotriazole (HOBt)) used with a dehydration condensation agent may be added. The dehydration condensation agent may be used in an amount of 0.5 to 5 equivalents, preferably 1 to 2 equivalents to 1 equivalent of the compound of the formula (II), and the additive may be used in an amount of 0.2 to 5 equivalents, preferably 1 to 2 equivalents to 1 equivalent of the compound of the formula (II). In Reaction 1-4, in a case where a base is used with the dehydration condensation agent, as the base, ones mentioned for the above Reaction B may be used. The base may be used in an amount of 0.5 to 10 equivalents, preferably 1 to 5 equivalents to the compound of the formula (II).

In Reaction 1-4, in a case where L in the compound of the formula (II-a) is an alkoxy, the compound of the formula (II) is reacted with an alkyl halide usually with the addition of a solvent and a base for esterification.

In Reaction 1-4, the alkyl halide may, for example, be methyl iodide or ethyl iodide. The alkyl halide may be used in an amount of 0.5 to 5 equivalents, preferably 0.8 to 1.5 equivalents to 1 equivalent of the compound of the formula (II).

In Reaction 1-4, as the base to be used for the reaction with the alkyl halide, ones mentioned for the above Reaction D may be used. The base may be used in an amount of 0.5 to 10 equivalents, preferably 1 to 5 equivalents to 1 equivalent of the compound of the formula (II).

In Reaction 1-4, in a case where L in the compound of the formula (II-a) is an alkylcarbonyloxy or an arylcarbonyloxy, the compound of the formula (II) is reacted with a carbonylation agent usually in the presence of a solvent and a base for carbonylation.

In Reaction 1-4, the carbonylation agent may, for example, be acetyl chloride, pivaloyl chloride, benzoyl chloride, acetic anhydride or benzoic anhydride. The carbonylation agent may be used in an amount of 0.5 to 10 equivalents, preferably 1 to 5 equivalents to 1 equivalent of the compound of the formula (II), or may be used in excess within a range not to impair the reaction. In a case where Reaction 1-4 is carbonylation, as the base used, ones mentioned for the above Reaction B may be used. The base may be used in an amount of 0.5 to 10 equivalents, preferably 1 to 5 equivalents to 1 equivalent of the compound of the formula (II).

In Reaction 1-4, the solvent may be any solvent so long as it is inert to the reaction, and one or more may properly be selected from aromatic hydrocarbons such as benzene, toluene, xylene and chlorobenzene; aliphatic hydrocarbons such as carbon tetrachloride, methyl chloride, chloroform, dichloromethane, dichloroethane, trichloroethane, hexane and cyclohexane; ethers such as dioxane, tetrahydrofuran, diethyl ether and dimethoxyethane; esters such as methyl acetate and ethyl acetate; aprotic polar solvents such as dimethyl sulfoxide, sulfolane, N,N-dimethylacetamide, N,N-dimethylformamide, N-methylpyrrolidone, pyridine, acetonitrile and propionitrile; ketones such as acetone and methyl ethyl ketone; protic polar solvents such as methanol and ethanol; water; and the like.

In Reaction 1-4, the reaction temperature is usually about -50°C to 200°C, preferably about -20°C to 100°C, and the reaction time is usually about 0.1 to 12 hours.

The compound of the formula (II) used in Reaction 1-3 and Reaction 1-4 may be produced in accordance with Reaction 1-1, Reaction 1-2 or a known method, or may be commercially available.

Reaction 1-5 may be carried out under conventional reaction conditions for protection of an amino group with a Boc group, for example, in accordance with the method described in Greene's PROTECTIVE GROUPS in ORGANIC SYNTHESIS (John Wiley and Sons, 2007, Peter G.M.Wuts, Theodora W.Greene).

The compound of the formula (VI) used in Reaction 1-5 may be produced by a known method, for example in accordance with the method described in WO2018/041563 or US Publication 2008/312255, or may be commercially available.

### [Reaction 2-1] to [Reaction 2-9]

Reaction 2-1 is a method of reacting a compound of the formula (10) with a compound of the formula (3-a) to obtain a compound of the formula (11). Reaction 2-2 is a method of reducing the compound of the formula (11) to obtain the compound of the formula (III). Reaction 2-1 and Reaction 2-2 may also be carried out continuously without isolating the compound of the formula (11).

Reaction 2-3 is a method of reacting a compound of the formula (11-a) with the compound of the formula (IV) to obtain a compound of the formula (11-b).

Reaction 2-4 is a method of reacting the compound of the formula (V) with a compound of the formula (3-a) to obtain the compound of the formula (III).

Reaction 2-5 is a method of obtaining the compound of the formula (10) from a compound of the formula (12) using a hydride reducing reagent. Reaction 2-6 is a method of reducing the compound of the formula (10) to obtain a compound of the formula (13). Reaction 2-7 is a method of obtaining the compound of the formula (V) from the compound of the formula (13) using a halogenating reagent or a sulfonylating reagent.

Reaction 2-8 is a method of obtaining a compound of the formula (V-b) from a compound of the formula (V-a) using a halogenating reagent. Reaction 2-9 is a method of obtaining a compound of the formula (V-b) from a compound of the formula (14) using a halogenating agent.

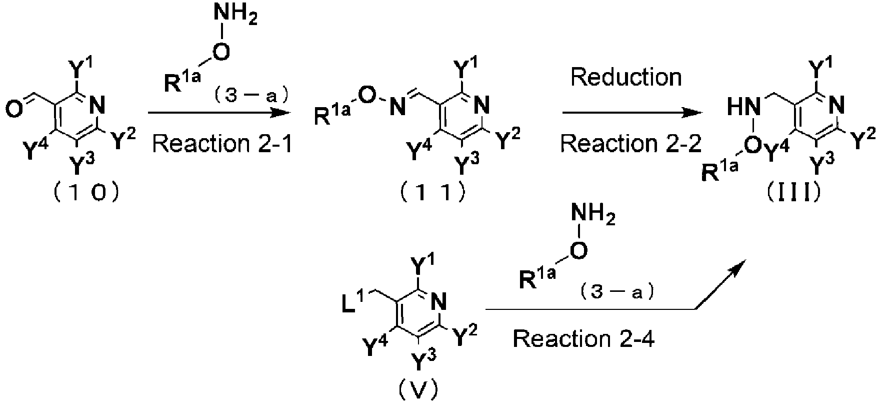

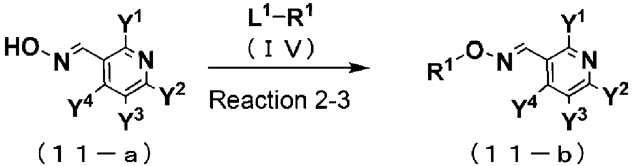

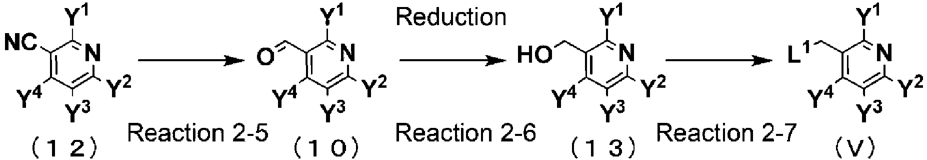

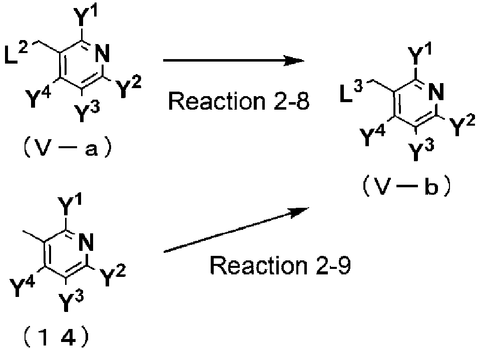

In the formulae in each Reaction, L² is a leaving group, such as trifluoromethanesulfonyloxy, methanesulfonyloxy or p-toluenesulfonyloxy, L³ is a halogen, and the other symbols are as defined above.

Reaction 2-1 may be carried out optionally with the addition of an acid, a base or a dehydrating agent. Reaction 2-1 may also be carried out in the presence of a solvent. In Reaction 2-1, the compound of the formula (3-a) may be used in an amount of 1 to 5 equivalents to 1 equivalent of the compound of the formula (10), or may be used in excess within a range not to impair the reaction. In Reaction 2-1, the compound of the formula (3-a) may be a salt of the compound of the formula (3-a) (for example, hydrochloride, sulfate or trifluoroacetate).

In Reaction 2-1, the acid may be either an inorganic acid or an organic acid, the inorganic acid may be hydrochloric acid, sulfuric acid or the like, and the organic acid may be acetic acid, methanesulfonic acid, p-toluenesulfonic acid or the like. The acid may be used in an amount of 0.1 to 10 equivalents to 1 equivalent of the compound of the formula (10), or may be used in excess within a range not to impair the reaction.

In Reaction 2-1, as the base, alkali metal alkoxides such as sodium methoxide, sodium ethoxide and potassium tert-butoxide; carbonates such as sodium carbonate and potassium carbonate; hydrogencarbonates such as sodium hydrogencarbonate and potassium hydrogencarbonate; metal hydroxides such as sodium hydroxide and potassium hydroxide; metal hydrides such as sodium hydride and potassium hydride; amines such as triethylamine and N,N-diisopropylethylamine; pyridines such as pyridine, 4-dimethylaminopyridine and 2,6-lutidine; alkali metal carboxylates such as sodium acetate and potassium acetate; and the like may be mentioned. The base may be used in an amount of 0.5 to 5 equivalents to 1 equivalent of the compound of the formula (3-a).

In Reaction 2-1, the dehydrating agent may, for example, be anhydrous magnesium sulfate, anhydrous sodium sulfate or molecular sieve.

In Reaction 2-1, the solvent may be any solvent so long as it is inert to the reaction, and one or more may properly be selected from aromatic hydrocarbons such as toluene and xylene; aliphatic hydrocarbons such as carbon tetrachloride, methyl chloride, chloroform, dichloromethane, dichloroethane, trichloroethane, hexane and cyclohexane; ethers such as dioxane, tetrahydrofuran, diethyl ether and dimethoxyethane; esters such as methyl acetate and ethyl acetate; aprotic polar solvents such as acetonitrile; protic polar solvents such as methanol and ethanol; water; and the like.

In Reaction 2-1, the reaction temperature is usually about -20°C to 200°C, preferably about 0°C to 150°C, and the reaction time is usually about 0.5 to 48 hours, preferably about 1 to 24 hours.

The compound of the formula (10) used in Reaction 2-1 may be produced in accordance with Reaction 2-5 or a known method, or may be commercially available.

Reaction 2-2 may be carried out usually in the presence of a reducing agent and a solvent, and optionally with the addition of an acid.

In Reaction 2-2, the reducing agent may, for example, be sodium borohydride, sodium cyanoborohydride, sodium triacetoxyborohydride or 2-picoline borane complex. The reducing agent may be used in an amount of 0.5 to 10 equivalents, preferably 1 to 5 equivalents to 1 equivalent of the compound of the formula (11). The acid may be either an inorganic acid or an organic acid, and the inorganic acid may be hydrochloric acid or the like, and the organic acid may be acetic acid, trifluoroacetic acid or the like. The acid may be used in an amount of 1 to 10 equivalents to 1 equivalent of the compound of the formula (11).

In Reaction 2-2, the solvent may be any solvent so long as it is inert to the reaction, and one or more may properly be selected from aromatic hydrocarbons such as benzene, toluene, xylene and chlorobenzene; aliphatic hydrocarbons such as carbon tetrachloride, methyl chloride, chloroform, dichloromethane, dichloroethane, trichloroethane, hexane and cyclohexane; ethers such as dioxane, tetrahydrofuran, diethyl ether and dimethoxyethane; esters such as methyl acetate and ethyl acetate; protic polar solvents such as methanol and ethanol; water; and the like.

In Reaction 2-2, the reaction temperature is usually about -20°C to 150°C, preferably about 0°C to 100°C, and the reaction time is usually about 0.5 to 48 hours, preferably about 1 to 24 hours.

Reaction 2-3 may be carried out in accordance with Reaction D. The compound of the formula (IV) may be used in an amount of 1 to 5 equivalents, preferably 1 to 2 equivalents to 1 equivalent of the compound of the formula (11-a). In Reaction 2-3, the base may be used in an amount of 1 to 10 equivalents, preferably 1 to 5 equivalent to 1 equivalent of the compound of the formula (11-a). In Reaction 2-3, the phase transfer catalyst may be used in an amount of 0.1 to 3 equivalents to 1 equivalent of the compound of the formula (11-a).

In Reaction 2-3, the reaction temperature is usually about -20°C to 150°C, preferably about 0°C to 100°C, and the reaction time is usually about 10 minutes to 48 hours, preferably about 1 to 24 hours.

Reaction 2-4 may be carried out in accordance with Reaction E. In Reaction 2-4, the compound of the formula (3-a) may be used in an amount of 1 to 5 equivalents, preferably 1 to 3 equivalents to 1 equivalent of the compound of the formula (V). In Reaction 2-4, the compound of the formula (3-a) may be a salt of the compound of the formula (3-a) (for example, hydrochloride, sulfate or trifluoroacetate). In Reaction 2-4, the base may be used in an amount of 1 to 10 equivalents, preferably 1 to 5 equivalents to 1 equivalent of the compound of the formula (V). In Reaction 2-4, the phase transfer catalyst may be used in an amount of 0.1 to 3 equivalents to 1 equivalent of the compound of the formula (V).

In Reaction 2-4, the reaction temperature is usually about -20°C to 150°C, preferably about 0°C to 100°C, and the reaction time is usually about 10 minutes to 48 hours, preferably about 1 to 24 hours.

The compound of the formula (V) used in Reaction 2-4 may be produced in accordance with a known method, Reaction 2-7, Reaction 2-8 or Reaction 2-9, or may be commercially available.

Reaction 2-5 may be carried out under conventional conditions for conversion of a cyano group into a formyl group, for example, in accordance with the method described in Journal of Medicinal Chemistry, 2008, 51, 4021-4029.

The compound of the formula (12) used in Reaction 2-5 may be produced in accordance with a known method, or may be commercially available.

Reaction 2-6 may be carried out under conventional conditions for reduction of a formyl group, for example, in accordance with the method described in Bioorganic & Medicinal Chemistry Letters, 2012, 22, 901-906.

Reaction 2-7 may be carried out under conventional conditions for halogenating a hydroxy group or under conventional conditions for sulfonylating a hydroxy group. As the respective conditions of Reaction 2-7, the method described in Journal of Medicinal Chemistry, 2003, 46, 453-456 or WO 2020/106307 may be mentioned.

Reaction 2-8 may be carried out under conventional conditions for halogenating a leaving group such as a mesyl group, for example, in accordance with the method described in Journal of Medicinal Chemistry, 2010, 53, 8421-8439.

The compound of the formula (V-a) used in Reaction 2-8 may be produced in accordance with Reaction 2-7 or a known method, or may be commercially available.

Reaction 2-9 may be carried out under conventional conditions for halogenating pyridylmethyl, for example, in accordance with the method described in Journal of Medicinal Chemistry, 2011, 54, 6106-6116.

The compound of the formula (14) used in Reaction 2-9 may be produced in accordance with a known method, or may be commercially available.

In the above Reaction, after completion of the reaction, conventional post-treatment (e.g. evaporation of the solvent, neutralization distillation, washing, extraction, or drying) may be carried out to obtain a desired compound. Such conventional post-treatment processes may be conducted alone or in combination of two or more processes, to isolate a desired compound. Further, after the post-treatment, as the case requires, the desired compound may be purified by a conventional method such as column chromatography or recrystallization. Each intermediate produced by the above Reaction may be subjected to the next step reaction as it is crude, without being isolated or purified.

The compound (I) is useful as an active ingredient of an agricultural and horticultural fungicide which can control harmful plant diseases at a low dose.

The compound (I) can control plant diseases caused by phytopathogen belonging to e.g. Oomycota, Endomyxa, Olpidiomycota, Ascomycota, Basidiomycota and Blastocladiomycota. It is particularly effective for controlling plant diseases caused by phytopathogen belonging to Oomycota, Endomyxa and Olpidiomycota.

As the above phytopathogen, the following may be mentioned.
Oomycota: phytopathogen belonging to Albuginales, Anisolpidiales, Lagenidiales, Leptomitales, Myzocytiopsidales, Olpidiopsidales, Peronosporales, Pythiales, Rhipidiales, Saprolegniales and Sclerosporales.
Endomyxa: phytopathogen belonging to Haplosporida, Paradiniida, Paramyxida, Gromiida, Phagomyxida, Plasmodiophorida and Vampyrellida.
Olpidiomycota: phytopathogen belonging to Olpidiales.
Ascomycota: phytopathogen belonging to Cladosporiales, Diaporthales, Erysiphales, Glomerellales, Helotiales, Hypocreales, Magnaporthales, Mycosphaerellales, Myriangiales, Pleosporales and Venturiales.
Basidiomycota: phytopathogen belonging to Agaricales, Cantharellales, Pucciniales and Ustilaginales.
Blastocladiomycota: phytopathogen belonging to Physodermatales.

As specific examples of the phytopathogen, the following may be mentioned.

Genus Phytophthora such as potato or tomato late blight (Phytophthora infestans), fig white powdery rot (Phytophthora palmivora), pear Phytophthora fruit rot or strawberry crown or root rot (Phytophthora cactorum), winter melon or pumpkin Phytophthora rot, sweet pepper or hot pepper Phytophthora blight (Phytophthora capsici), tomato gray late blight (Phytophthora capsici), eggplant or watermelon brown rot (Phytophthora capsici), citrus brown rot (Phytophthora citricola), adzuki bean stem rot (Phytophthora vignae f. sp. adzukicola), Edamame stem rot (Phytophthora megasperma var. sojae) and onion or allium chinense white tip (Phytophthora porri); genus Pseudoperonospora such as cucumber, pumpkin, melon or zucchini downy mildew (Pseudoperonospora cubensis) and hop downy mildew (Pseudoperonospora humuli); genus Plasmopara such as grape downy mildew (Plasmopara viticola) and Japanese honeywort downy mildew (Plasmopara nivea); genus Hyaloperonospora such as cabbage or Chinese cabbage downy mildew (Hyaloperonospora brassicae); genus Bremia such as lettuce downy mildew (Bremia lactucae); genus Pythium such as rice seedling blight (Pythium graminicola), wheat browning root rot (Pythium iwayamai), Chinese cabbage Pythium rot (Pythium aphanidermatum), Japanese ginger rhizome rot (Pythium zingiberis) and ginger rhizome rot (Pythium ultimum var. ultimum); genus Aphanomyces such as Japanese radish black-root (Aphanomyces raphanin) and spinach root rot (Aphanomyces cochlioides); genus Albugo such as spinach, turnip, Japanese radish or turnip rape white rust (Albugo macrospora), wasabi white rust (Albugo wasabiae), and water spinach white rust (Albugo ipomoeae-aquaticae); and genus Peronospora such as soybean downy mildew (Peronospora manshurica), broccoli or turnip downy mildew (Peronospora parasitica), leek, tree onion or onion downy mildew (Peronospora destructor), spinach downy mildew (Peronospora farinosa f. sp. Spinaciae) and basil downy mildew (Peronospora belbahrii).

Genus Plasmodiophora such as Chinese cabbage, cabbage, cauliflower, turnip rapa, broccoli or turnip clubroot (Plasmodiophora brassicae); genus Polymyxa such as Polymyxa betae transmitting sugar beet rhizomania (Beet necrotic yellow vein virus); genus Spongospora such as potato powdery scab (Spongospora subterranea); and genus Olpidium such as Olpidium virulentus transmitting lettuce big vein virus (Mirafiori lettuce bigvein virus).

Genus Erysiphe such as wheat powdery mildew (Erysiphe graminis); genus Setosphaeria such as corn leaf blight (Setosphaeria turcica); genus Sphaerotheca such as cucumber powdery mildew (Sphaerotheca fuliginea), and strawberry powdery mildew (Sphaerotheca humuli); genus Uncinula such as grape powdery mildew (Uncinula necator); genus Podosphaera such as apple powdery mildew (Podosphaera leucotricha); genus Mycosphaerella such as pea Mycosphaerella blight (Mycosphaerella pinodes), apple black spot (Mycosphaerella pomi), banana black sigatoka (Mycosphaerella musicola), Japanese persimmon circular leaf spot (Mycosphaerella nawae), and strawberry leaf spot (Mycosphaerella fragariae); genus Venturia such as apple scab (Venturia inaequalis) and pear scab (Venturia nashicola); genus Pyrenophora such as barley net blotch (Pyrenophora teres) and barley stripe (Pyrenophora graminea); and genus Sclerotinia such as kidney bean stem rot, cucumber, cabbage, Chinese cabbage, hot pepper or sweet pepper sclerotinia rot or onion watery soft rot (Sclerotinia sclerotiorum), wheat Sclerotinia snow blight (Sclerotinia borealis), tomato white mold (Sclerotinia minor), and alfalfa Sclerotinia root and crown rot (Sclerotinia trifoliorum).

Genus Botryotinia such as peanut small sclerotinia rot (Botryotinia arachidis); genus Cochliobolus such as rice brown spot (Cochliobolus miyabeanus); genus Didymella such as cucumber gummy stem blight (Didymella bryoniae); genus Gibberella such as rice bakanae (Gibberella fujikuroi); genus Elsinoe such as grape anthracnose (Elsinoe ampelina) and citrus scab (Elsinoe fawcettii); genus Diaporthe such as citrus melanose (Diaporthe citri) and grape swelling arm (Diaporthe sp.); genus Monilinia such as apple Monilia leaf blight (Monilinia mali) and peach brown rot (Monilinia fructicola), and genus Glomerella such as grape ripe rot (Glomerella cingulata).

Genus Rhizoctonia such as rice sheath blight (Rhizoctonia solani); genus Ustilago such as wheat loose smut (Ustilago nuda); genus Puccinia such as oat crown rust (Puccinia coronata), wheat brown rust (Puccinia recondita), and wheat yellow rust (Puccinia striiformis); genus Phakopsora such as Asian soybean rust (Phakopsora pachyrhizi); and genus Typhula such as wheat or barley snow mold (Typhula incarnata and Typhula ishikariensis).

Genus Septoria such as wheat Septoria nodorum blotch (Septoria nodorum) and wheat Septoria tritici blotch (Septoria tritici); genus Botrytis such as grape, citrus, cucumber, tomato, strawberry, eggplant, kidney bean, adzuki bean, pea, peanut, hot pepper, sweet pepper, lettuce, onion, statice, carnation, pansy or sunflower gray mold or rose Botrytis blight (Botrytis cinerea), onion gray mold (Botrytis allii), and onion leaf blight or leaf spot (Botrytis squamosa, Botrytis byssoidea and Botrytis tulipae); genus Fusarium such as wheat head blight (Fusarium graminearum) and cucumber Fusarium wilt (Fusarium oxysporum); genus Pyricularia such as rice blast (Pyricularia oryzae); genus Cercospora such as sugar beet Cercospora leaf spot (Cercospora beticola), and Japanese persimmon Cercospora leaf spot (Cercospora kakivora); genus Colletotrichum such as cucumber anthracnose (Colletotrichum orbiculare), and coffee berry disease (Colletotrichum coffeanum); genus Alternaria such as apple Alternaria blotch (Alternaria alternata apple pathotype), pear black spot (Alternaria alternata Japanese pear pathotype), potato or tomato early blight (Alternaria solani), cabbage or Chinese cabbage Alternaria leaf spot (Alternaria brassicae), cabbage dark leaf spot (Alternaria brassicicola) and onion or leek purple blotch (Alternaria porri); genus Phoma such as cabbage black leg (Phoma lingam); genus Pseudocercosporella such as wheat eyespot (Pseudocercosporella herpotrichoides); genus Pseudocercospora such as grape isariopsis leaf spot (Pseudocercospora vitis); genus Rhynchosporium such as barley scald (Rhynchosporium secalis); genus Cladosporium such as peach scab (Cladosporium carpophilum); genus Phomopsis such as peach Phomopsis rot (Phomopsis sp.); genus Gloeosporium such as Japanese persimmon anthracnose (Gloeosporium kaki); genus Fulvia such as tomato leaf mold (Fulvia fulva); genus Corynespora such as cucumber target spot (Corynespora cassiicola); and genus Physoderma such as corn brown spot (Physoderma maydis).

The compound (I) can control the above various phytopathogens, and thus can control various diseases preventively or curatively. Particularly, the compound (I) is effective for controlling plant diseases which are problematic in agricultural and horticultural fields, including diseases of Gramineae crops, such as diseases of rice, such as damping-off caused by Pythium, blast caused by Pyricularia, Bakanae disease caused by Fusarium, brown spot caused by Cochliobolus, and sheath blight caused by Rhizoctonia; diseases of wheat and the like such as powdery mildew caused by Erysiphe, scab or crown rot caused by Fusarium, leaf rust caused by Puccinia, browning root rot caused by Pythium, loose smut caused by Ustilago, eyespot caused by Pseudocercosporella, and speckled leaf blotch or glume blotch caused by Septoria; diseases of corn such as Gibberella ear rot caused by Fusarium, brown spot caused by Physoderma, leaf rust caused by Puccinia, leaf blight caused by Setosphaeria, leaf spot caused by Cochliobolus, browning root rot caused by Pythium, and smut caused by Ustilago; and diseases of sugar cane such as smut caused by Ustilago, leaf scorch caused by Stagonospora, leaf rust caused by Puccinia, top rot caused by Gibberella, sooty mold caused by Caldariomyces, and leaf blight caused by Pseudocercosporella; diseases of Leguminosae crops, such as powdery mildew caused by Oidium, rust caused by Phakopsora, downy mildew caused by Peronospora, leaf blight or root and stem rot caused by Phytophthora, anthracnose caused by Colletotrichum, stem rot caused by Sclerotinia, gray mold caused by Botrytis, and root rot or foot rot caused by Fusarium; diseases of Brassicaceae crops, such as yellows caused by Fusarium, downy mildew caused by Peronospora or Hyaloperonospora, Alternaria leaf spot caused by Alternaria, root rot caused by Phoma, clubroot caused by Plasmodiophora, black root caused by Aphanomyces, and Pythium rot caused by Pythium; diseases of Asteraceae crops, such as downy mildew caused by Bremia, Phytophthora rot caused by Phytophthora, Botrytis blight caused by Botrytis, stem rot caused by Sclerotinia, and rust caused by Aecidium; diseases of Solanaceae crops, such as diseases of tomato such as early blight caused by Alternaria, leaf mold caused by Fulvia, late blight or Phytophthora blight caused by Phytophthora, gray mold caused by Botrytis, powdery mildew caused by Oidium, Fusarium wilt caused by Fusarium, and Cercospora leaf mold caused by Pseudocercosporella; and diseases of potato such as early blight caused by Alternaria, late blight or tuber rot caused by Phytophthora, Sclerotial rot caused by Sclerotinia, and dry rot caused by Fusarium; diseases of Cucurbitaceae crops, such as anthracnose caused by Colletotrichum, powdery mildew caused by Sphaerotheca, gummy stem blight caused by Didymella, downy mildew caused by Pseudoperonospora, Phytophthora rot or brown rot caused by Phytophthora, Corynespora leaf spot caused by Corynespora, and Fusarium wilt caused by Fusarium; diseases of Amaryllidaceae allium crops, such as downy mildew caused by Peronospora, Phytophthora rot caused by Phytophthora, gray mold caused by Botrytis, neck rot caused by Sclerotinia, and rust caused by Puccinia; diseases of Umbelliferae crops, such as downy mildew caused by Plasmopara, leaf blight or Alternaria black rot caused by Alternaria, gray mold caused by Botrytis, Sclerotinia rot caused by Sclerotinia, powdery mildew caused by Erysiphe, and leaf spot caused by Cercospora; diseases of Liliaceae crops, such as Botrytis blight caused by Botrytis, Phytophthora blight caused by Phytophthora, and stem blight caused by Phomopsis; diseases of Polygonaceae crops, such as downy mildew caused by Peronospora, powdery mildew caused by Erysiphe, and damping-off caused by Rhizoctonia; diseases of Convolvulaceae crops, such as white rust caused by Albugo, stem rot caused by Fusarium, black rot caused by Ceratocystis, and soil rot caused by Streptomyces; diseases of Chenopodiaceae crops, such as root rot caused by Aphanomyces, white rust caused by Albugo, downy mildew caused by Peronospora, late blight caused by Phytophthora, gray mold caused by Botrytis, root rot caused by Sclerotinia, powdery mildew caused by Oidium, and brown spot caused by Cercospora; diseases of Vitaceae crops, such as anthracnose caused by Elsinoe, ripe rot caused by Colletotrichum, powdery mildew caused by Erysiphe, downy mildew caused by Plasmopara, gray mold caused by Botrytis, leaf blight caused by Pseudocercospora, and swelling arm caused by Diaporthe; diseases of Rosaceae crops, such as diseases of strawberry, such as powdery mildew caused by Sphaerotheca, gray mold caused by Botrytis, crown rot caused by Glomerella, and crown and root rot caused by Fusarium; diseases of apple, such as blossom blight caused by Monilinia, powdery mildew caused by Podosphaera, Alternaria blotch caused by Alternaria, scab caused by Venturia, bitter rot caused by Glomerella, blotch caused by Diplocarpon, ring rot caused by Botryosphaeria, fly speck caused by Zygophiala, sooty blotch caused by Gloeodes, and fruit spot caused by Mycosphaerella; diseases of pear, such as scab caused by Venturia, black spot caused by Alternaria, powdery mildew caused by Phyllactinia, Phytophthora fruit rot caused by Phytophthora, and Fusarium fruit rot caused by Fusarium; and diseases of peach, such as brown rot caused by Monilinia, scab caused by Cladosporium, and Phomopsis rot caused by Phomopsis; diseases of Rutaceae crops, such as diseases of citrus, such as melanose caused by Diaporthe, scab caused by Elsinoe, and Fusarium wilt caused by Fusarium; diseases of Ebenaceae crops, such as anthracnose caused by Gloeosporium, leaf spot caused by Cercospora, powdery mildew caused by Phyllactinia, and fly speck caused by Zygophiala; diseases of Theaceae crops, such as anthracnose caused by Colletotrichum, gray mold caused by Pestalotiopsis, bacterial shoot blight caused by Pseudomonas, and leaf and stem gall caused by Exobasidium; diseases of Zingiberaceae crops, such as root rot caused by Pythium; diseases of Cannabaceae crops, such as downy mildew caused by Pseudoperonospora; and diseases of Lamiaceae crops, such as downy mildew caused by Peronospora.

The compound (I) is effective also for seed borne diseases, including diseases of Gramineae crops, such as diseases of wheat and the like, such as scab or crown rot caused by Fusarium, anthracnose caused by Colletotrichum, bunt caused by Tilletia, loose smut caused by Ustilago, Cephalosporium stripe caused by Cephalosporium, and glume blotch caused by Septoria; diseases of corn, such as leaf spot caused by Bipolaris, anthracnose caused by Colletotrichum, and seedling blight caused by Fusarium; and diseases of sugar cane, such as red rot caused by Glomerella, pineapple disease caused by Ceratocysti, and downy mildew caused by Sclerospora; diseases of Leguminosae crops, such as diseases of soybean, such as purple stain caused by Cercospora, downy mildew caused by Peronospora, Fusarium blight caused by Fusarium, Septoria brown spot caused by Septoria, pod and stem blight caused by Diaporthe, anthracnose caused by Colletotrichum, and sleeping-blight caused by Septogloeum; diseases of Brassicaceae crops, such as diseases of cabbage, such as Alternaria leaf spot or Alternaria sooty spot caused by Alternaria, downy mildew caused by Peronospora, bacterial leaf spot caused by Pseudomonas, black root caused by Xanthomonas, and black leg caused by Phoma; diseases of Japanese radish, such as Alternaria leaf spot caused by Alternaria, yellows caused by Fusarium, and black rot caused by Xanthomonas; and diseases of Chinese cabbage, such as Alternaria leaf spot caused by Alternaria, black rot caused by Xanthomonas, and yellows caused by Verticillium; diseases of Solanaceae crops, such as diseases of tomato, such as early blight caused by Alternaria, bacterial canker caused by Clavibacter, and bacterial spot caused by Xanthomona; diseases of eggplant, such as early blight caused by Alternaria, and brown spot caused by Phomopsis; and diseases of potato, such as scab caused by Streptomyces, silver scurf caused by Helminthosporium, and powdery scab caused by Spongospora; diseases of Cucurbitaceae crops, such as diseases of cucumber, such as leaf blight caused by Alternaria, bacterial spot caused by Pseudomonas, and bacterial brown spot caused by Xanthomonas; diseases of Amaryllidaceae allium crops, such as Alternaria leaf spot caused by Alternaria, gray-mold neck rot or mycelial rot caused by Botrytis, dry rot caused by Fusarium, downy mildew caused by Peronospora, and leaf blight or Phytophthora rot caused by Phytophthora; diseases of Umbelliferae crops, such as diseases of carrot, such as leaf blight or Alternaria black rot caused by Alternaria, and bacterial blight caused by Xanthomonas; and diseases of celery, such as late blight caused by Septoria, stem rot caused by Sclerotinia, and bacterial leaf blight caused by Pseudomonas; and diseases of Chenopodiaceae crops, such as diseases of spinach, such as downy mildew caused by Peronospora, Fusarium wilt caused by Fusarium, and anthracnose caused by Colletotrichum.

The compound (I) is effective also for controlling soil diseases caused by phytopathogen such as Fusarium, Pythium, Rhizoctonia, Verticillium, Plasmodiophora, and Thielaviopsis.

The compound (I) can control the above various diseases preventively or curatively. By test methods disclosed in the following Examples, certain Invention Compounds (I) exhibit excellent preventive or curative controlling effects at low dose (for example, 100 ppm, 25 ppm, 12.5 ppm, 6.3 ppm, 3.1 ppm, 1.6 ppm, 0.8 ppm, 0.4 ppm, 0.2 ppm or 0.1 ppm).

The compound (I) has excellent rainfastness, residual effects and penetration effects, and thus by applying the compound (I) to a plant body, it is possible to control harmful fungi on above-ground parts of the plant for a certain period.

For controlling various harmful plant diseases, an effective amount of the compound (I) may be applied to a plant body, phytopathogen or soil.

The effective amount means an application amount of the compound (I) at which the compound (I) exhibits a control effect against various harmful plant diseases.

The plant body means above-ground parts such as trunks, stems, leaves, flowers, heads and fruits, below-ground parts such as tubers, rhizomes and roots, and seeds of plant, seedlings, and transplanted seedlings, etc.

The soil means a field where plants are grown, including e.g. agricultural fields such as crop plant fields, paddy fields and orchards, and non-agricultural fields such as lawn and forest land.

The plants to which the compound (I) is applied are not particularly limited so long as they are agriculturally or horticulturally useful plants, and their examples include Gramineae crops (such as rice, wheat, barley, oat, rye, corn and sugar cane), Leguminosae crops (such as soybean, kidney bean, adzuki bean, pea, peanut, Edamame and alfalfa), Brassicaceae crops (such as cabbage, Chinese cabbage, Japanese radish, turnip, broccoli, cauliflower, Turnip rape, rapeseed and horseradish), Asteraceae crops (such as lettuce, burdock, crown daisy and sunflower), Solanaceae crops (such as potato, eggplant, tomato, sweet pepper, tobacco and hot pepper), Cucurbitaceae crops (such as cucumber, pumpkin, melon, watermelon, winter melon and zucchini), Amaryllidaceae allium crops (such as leek, chinese chive, allium chinense, garlic, onion and shallot), Umbelliferae crops (such as celery, carrot, parsley and Japanese honeywort), Liliaceae crops (such as lily, tulip and asparagus), Polygonaceae crops (such as buckwheat), Convolvulaceae crops (such as sweet potato and water spinach), Chenopodiaceae crops (such as spinach and sugar beet), Vitaceae crops (such as grape), Rosaceae crops (such as rose, strawberry, apple, pear, peach, loquat and almond), Rutaceae crops (such as mandarin orange, lemon, orange and citrus), Ebenaceae crops (such as Japanese persimmon), Moraceae crops (such as fig), Theaceae crops (such as tea), Oleaceae crops (such as olive and jasmine), Malvaceae crops (such as cotton, cacao and okra), Musaceae crops (such as banana), Zingiberaceae crops (such as ginger and Japanese ginger), Cannabaceae crops (such as hop), Lamiaceae crops (such as basil), Rubiaceae crops (such as coffee plant), Bromeliaceae crops (such as pineapple and ananas), Plumbaginaceae crops (such as statice), Caryophyllaceae crops (such as carnation), and Violaceae crops (such as pansy).

The plants include plants generated by gene recombination or gene editing, for example, plants having imparted resistance to environmental stress, resistance to herbicides, resistance to pests and resistance to phytopathogen, and plants having growth, fertility, quality or yield of crops modified.

The compound (I) may be obtained usually by mixing the compound (I) with additives and applied in the form of various formulations such as dusts, granules, water dispersible granules, wettable powders, water-based suspensions, oil-based suspensions, water soluble powders, emulsifiable concentrates, liquid, pastes, aerosols, ultra low-volume formulations and microcapsules. It may be formed into any usual formulation used in this field, so long as the object of the present invention is thereby met. The additives used for formulation may be a solid carrier or a liquid carrier and as the case requires, a surfactant and other additives for formulation may be used.

Specific examples of the solid carrier include diatomaceous earth, slaked lime, calcium carbonate, talc, white carbon, kaoline, bentonite, kaolinite, sericite, clay, sodium carbonate, sodium bicarbonate, salt cake, zeolite, starch and pulverized silica.

Specific examples of the liquid carrier include water, toluene, xylene, solvent naphtha, dioxane, acetone, isophorone, methyl isobutyl ketone, chlorobenzene, cyclohexane, dimethyl sulfoxide, N,N-dimethylformamide, N,N-dimethylacetamide, N-methyl-2-pyrrolidone and an alcohol.

Specific examples of the surfactant include anionic surfactants and spreaders such as a salt of fatty acid, a benzoate, an alkylsulfosuccinate, a dialkylsulfosuccinate, a polycarboxylate, a salt of alkylsulfuric acid ester, an alkyl sulfate, an alkylaryl sulfate, an alkyl diglycol ether sulfate, a salt of alcohol sulfuric acid ester, an alkyl sulfonate, an alkylaryl sulfonate, an aryl sulfonate, a lignin sulfonate, an alkyldiphenyl ether disulfonate, a polystyrene sulfonate, a salt of alkylphosphoric acid ester, an alkylaryl phosphate, a styrylaryl phosphate, a salt of polyoxyethylene alkyl ether sulfuric acid ester, a polyoxyethylene alkylaryl ether phosphate, a polyoxyethylene alkylaryl ether sulfate, a salt of polyoxyethylene alkylaryl ether sulfuric acid ester, a polyoxyethylene alkyl ether phosphate, a salt of polyoxyethylene alkylaryl phosphoric acid ester and a salt of naphthalene sulfonate condensed with formaldehyde; and nonionic surfactants and spreaders such as a sorbitan fatty acid ester, a glycerin fatty acid ester, a fatty acid polyglyceride, a fatty acid alcohol polyglycol ether, acetylene glycol, acetylene alcohol, an oxyalkylene block polymer, a polyoxyethylene alkyl ether, a polyoxyethylene alkylaryl ether, a polyoxyethylene styrylaryl ether, a polyoxyethylene glycol alkyl ether, a polyethylene glycol, a polyoxyethylene fatty acid ester, a polyoxyethylene sorbitan fatty acid ester, a polyoxyethylene glycerin fatty acid ester, a polyoxyethylene hydrogenated castor oil and a polyoxypropylene fatty acid ester.

Specific examples of other additives for formulation include vegetable oils and mineral oils such as olive oil, kapok oil, castor oil, palm oil, camellia oil, coconut oil, sesame oil, corn oil, rice bran oil, peanut oil, cottonseed oil, soybean oil, rapeseed oil, linseed oil, tung oil and liquid paraffins; silicone; and xanthan gum.

These additive components may suitably be selected for use alone or in combination as a mixture of two or more of them, so long as the object of the present invention is met. Further, in addition to the above, various additives known in this technical field, such as a filler, a thickener, an anti-settling agent, an anti-freezing agent, a dispersion stabilizer, a safener and an anti-mold agent, may be used. The mixing ratio of the compound (I) to such various additives is usually 0.001:99.999 to 95:5, preferably 0.005:99.995 to 90:10, by weight ratio. In the actual application of such a formulation, it may be used as it is, or may be diluted to a predetermined concentration with a diluent such as water, and may be mixed with various spreaders (e.g. surfactants, vegetable oils or mineral oils), as the case requires.

The application of the compound (I) cannot generally be defined, as it varies depending upon the weather conditions, the type of the formulation, the plants to be treated, the application season, the application site, the type or degree of outbreak of harmful plant diseases, etc. However, an effective amount of the compound (I) may be applied by a conventional application method, that is spraying, soil treatment, seed treatment, etc. When an effective amount of the compound (I) is applied by a conventional application method, it is possible to apply the compound (I) at an active ingredient concentration of 0.1 to 10,000 ppm, preferably 1 to 2,000 ppm, more preferably 1 to 1,000 ppm.

In a conventional application method, a composition containing the compound (I) as an active ingredient (hereinafter sometimes referred to as the present composition) or a diluted product thereof may also be applied. In the case of spraying, the application amount is such that the amount of the present composition is about 10 g to 100,000 g, per hectare. In the case of soil treatment, the application amount is such that the amount of the present composition is about 0.01 g to 1,000 g, per hectare. In the case of seed treatment, the application amount is such that the amount of the present composition is about 0.001 g to 100 g, preferably about 0.01 g to 1 g per 1 kg of seeds.

The spraying is an application method of spraying an effective amount of the present composition to the surface of the trunk, bud, stem, leaf, flower, head or fruit of a plant, or to phytopathogen for controlling the phytopathogen. For example, foliar spraying, trunk spraying, etc. may be mentioned.

The soil treatment is to apply the present composition to soil so that an effective amount of the compound (I) penetrates into the plant body e.g. from the root of the plant, for protecting crops from being damaged by phytopathogen. The soil application includes, for example, irrigation application (the present composition is applied by irrigation to soil in which a plant is to be grown), soil mixing application (soil in which a plant is to be grown and the present composition are mixed), planting hole application, application to the plant foot or to soil between plant feet (by spraying or irrigation), and mixing with culture soil or soil to be used for a nursery box, a nursery tray, a paper pot or a seed bed.

The seed treatment is to apply the present composition to seeds, bulbs or the like of a crop plant directly or to the vicinity thereof, to control phytopathogen, for protecting crops from being damaged by phytopathogen. For example, coating, dusting or dipping treatment may be mentioned.

Further, seedling treatment (by irrigation or dipping), dipping treatment of bulbs, tubers, scaly bulbs, roots or the like in the present composition, and hydroponics in which the present composition is mixed with a hydroponics culture medium, may be mentioned. The application may be to the whole plant or to a part of a plant (e.g. stem, leaf, bud, flower, head, fruit, trunk, seed, bulb, tuber, scaly bulb or root).

The present composition may be mixed with or may be used in combination with other components selected from other agricultural and horticultural chemicals, fertilizers and safeners, whereby more excellent effects or activities may sometimes be obtained.

Mixing with or use in combination with other components means that the present composition and other components are used simultaneously, separately or with an interval.

Such other agricultural and horticultural chemicals include, for example, a herbicide, an insecticide, a miticide, a nematicide, a soil pesticide, a fungicide, an antivirus agent, an attractant, an antibiotic, a plant hormone, a plant growth regulator, etc. Especially, a mixed fungicidal composition having the present composition mixed with or used in combination with one or more active ingredient compounds of other fungicide, may improve the application range, the application time, the controlling activities, etc. to preferred directions. The present composition and the active ingredient compounds of other fungicide may separately be formulated so that they are mixed for use at the time of application, or they may be formulated together. The present invention includes such a mixed fungicidal composition.

The mixing ratio of the compound (I) to the active ingredient compounds of other fungicide can not generally be defined as it varies depending upon the weather conditions, the types of formulations, the crop plants to be treated, the application time, the application site, the types or degree of outbreak of harmful plant diseases, etc., but it is usually within a range of 1:300 to 300:1, preferably 1:100 to 100:1, by weight ratio. Further, the dose for the application is such that the total amount of the active ingredient compounds is 0.1 to 70,000 g, preferably 1 to 30,000 g, per hectare. The present invention includes a method for controlling harmful plant diseases by application of such a mixed fungicidal composition.

When the present composition is applied, it may be used in combination with other agricultural and horticultural chemicals, such as a fungicide, an insecticide, a miticide, a nematicide, a soil insect pesticide, an antivirus agent, an attractant, a herbicide and a plant growth regulator.

The active ingredient compounds of a fungicide in the above-mentioned other agricultural and horticultural chemicals may properly be selected, for example, from the following group of compounds (by common names). In a case where these compounds have their salts, alkyl esters, various structural isomers such as optical isomers, etc., all of them are included, even if no specific disclosure thereof is made.

Anilinopyrimidine compounds such as mepanipyrim, pyrimethanil and cyprodinil;
triazolopyrimidine compounds such as ametoctradin;
triazolobenzothiazole compounds such as tricyclazole;
pyridinamine compounds such as fluazinam;
azole compounds such as triadimefon, bitertanol, triflumizole, etaconazole, propiconazole, penconazole, flusilazole, myclobutanil, cyproconazole, tebuconazole, hexaconazole, furconazole-cis, prochloraz, metconazole, epoxiconazole, tetraconazole, oxpoconazole fumarate, prothioconazole, triadimenol, flutriafol, difenoconazole, fluquinconazole, fenbuconazole, bromuconazole, diniconazole, simeconazole, pefurazoate, ipconazole, imibenconazole, azaconazole, triticonazole, imazalil, ipfentrifluconazole and mefentrifluconazole;
quinoxaline compounds such as chinomethionat;
dithiocarbamate compounds such as maneb, zineb, mancozeb, polycarbamate, metiram, propineb and thiram;
organic chlorine compounds such as phthalide, chlorothalonil and quintozene;
imidazole compounds such as benomyl, thiophanate-methyl, carbendazim, thiabendazole and fuberiazole;
cyanoacetamide compounds such as cymoxanil;
acylamino acid compounds such as metalaxyl, metalaxyl-M (another name: mefenoxam), oxadixyl, ofurace, benalaxyl, benalaxyl-M (another name: kiralaxyl, chiralaxyl), furalaxyl and valifenalate;
anilide compounds such as cyprofuram, carboxin, oxycarboxin, thifluzamide, boscalid, fenhexamid, isotianil, tiadinil and pyraziflumid;
sulfamide compounds such as dichlofluanid;
copper compounds such as cupric hydroxide, oxine copper, anhydrous copper sulfate, copper nonylphenolsulfonate, copper 8-hydroxyquinoline and dodecylbenzenesulfonic acid bisethylenediamine copper(II) complex salt (another name: DBEDC);
organophosphorus compounds such as fosetyl-Al, tolclofos-Methyl, edifenphos, and iprobenfos;
phthalimide compounds such as captan, captafol and folpet;
dicarboxyimide compounds such as procymidone, iprodione and vinclozolin;
benzanilide compounds such as flutolanil, mepronil, benodanil and flufenoxadiazam;
amide compounds such as carpropamid, diclocymet, silthiofam and fenoxanil;
pyrazole carboxamide compounds such as benzovindiflupyr, bixafen, fluindapyr, fluxapyroxad, furametpyr, isopyrazam, penflufen, penthiopyrad, pydiflumetofen, sedaxane, isoflucypram, inpyrfluxam, pyrapropoyne and fenopyramid;
benzamide compounds such as fluopicolide, fluopyram, zoxamide and fluopimomide;
flanilide compounds such as fenfuram;
thiophene amide compounds such as isofetamide;
piperazine compounds such as triforine;
pyridine compounds such as pyrifenox, pyrisoxazole and aminopyrifen;
pyrimidine compounds such as fenarimol, ferimzone, nuarimol and flumetylsulforim;
piperidine compounds such as fenpropidin;
morpholine compounds such as fenpropimorph and tridemorph;
organotin compounds such as fentin hydroxide and fentin acetate;
urea compounds such as pencycuron;
carboxylic acid amide compounds such as dimethomorph, flumorph, pyrimorph, iprovalicarb, benthiavalicarb-isopropyl and mandipropamid;
phenyl carbamate compounds such as diethofencarb;
cyanopyrrole compounds such as fludioxonil and fenpiclonil;
strobilurin compounds such as azoxystrobin, kresoxim-methyl, metominostrobin, trifloxystrobin, picoxystrobin, oryzastrobin, dimoxystrobin, pyraclostrobin, fluoxastrobin, Pyraoxystrobin, Pyrametostrobin, coumoxystrobin, enoxastrobin, fenaminstrobin, flufenoxystrobin, triclopyricarb, mandestrobin;
oxazole compounds such as famoxadone and oxathiapiprolin;
thiazolecarboxamide compounds such as ethaboxam;
imidazolinone compounds such as fenamidone;
benzenesulfonamide compounds such as flusulfamide;
oxime ether compounds such as cyflufenamid;
anthraquinone compounds such as dithianon;
crotonic acid compounds such as meptyldinocap;
antibiotics such as validamycin, kasugamycin, streptomycin and polyoxins;
guanidine compounds such as iminoctadine, dodine and guazatine;
aliphatic nitrogen compounds such as butylamine and seboctylamine;
quinoline compounds such as tebufloquin, quinoxyfen, quinofumelin, ipflufenoquin and feneptamidoquin;
thiazolidine compounds such as flutianil;
carbamate compounds such as propamocarb hydrochloride, pyribencarb and tolprocarb;
tetrazole compounds such as picarbutrazox and metyltetraprole;
sulfonamide compounds such as amisulbrom and cyazofamid;
allyl phenyl ketone compounds such as metrafenone and pyriofenone;
benzothiazole compounds such as probenazole and dichlobentiazox;
phenylpyrazole compounds such as fenpyrazamine;
dithiolane compounds such as isoprothiolane;
picolinamide compounds such as fenpicoxamid and florylpicoxamid;
sulfur compounds such as sulfur and lime sulfur;
other compounds such as pyroquilon, diclomezine, chloropicrin, dazomet, metam-sodium, proquinazid, spiroxamine and dipymetitrone;
microbial fungicides such as Bacillus amyloliqefaciens strain QST713, Bacillus amyloliqefaciens strain FZB24, Bacillus amyloliqefaciens strain MBI600, Bacillus amyloliqefaciens strain D747, Pseudomonas fluorescens, Bacillus subtilis and Trichoderma atroviride SKT-1; and
plant extracts such as tea tree oil.

The active ingredient compounds of an insecticide, a nematicide, a miticide or a soil pesticide, in the above-mentioned other agricultural and horticultural chemicals may properly be selected, for example, from the following group of compounds (by common names). In a case where these compounds have their salts, alkyl esters, various structural isomers such as optical isomers, etc., all of them are included, even if no specific disclosure thereof is made.
Organic phosphoric acid ester compounds such as profenofos, dichlorvos, fenamiphos, fenitrothion, EPN ((RS)-(O-ethyl O-4-nitrophenyl phenylphosphonothioate)), diazinon, chlorpyrifos, chlorpyrifos-methyl, acephate, prothiofos, fosthiazate, cadusafos, disulfoton, isoxathion, isofenphos, ethion, etrimfos, quinalphos, dimethylvinphos, dimethoate, sulprofos, thiometon, vamidothion, pyraclofos, pyridaphenthion, pirimiphos-methyl, propaphos, phosalone, formothion, malathion, tetrachlorvinphos, chlorfenvinphos, cyanophos, trichlorfon, methidathion, phenthoate, oxydeprofos, (another name ESP), azinphos-methyl, fenthion, heptenophos, parathion, phosphocarb, demeton-S-methyl, monocrotophos, methamidophos, imicyafos, parathion-methyl, terbufos, phosphamidon, phosmet and phorate;
carbamate compounds such as carbaryl, propoxur, aldicarb, carbofuran, thiodicarb, methomyl, oxamyl, ethiofencarb, pirimicarb, fenobucarb, carbosulfan, benfuracarb, bendiocarb, furathiocarb, isoprocarb, metolcarb, xylylcarb, XMC (3,5-xylyl methylcarbamate) and fenothiocarb;
nereistoxin derivatives such as cartap, thiocyclam, thiocyclam oxalate, thiocyclam hydrochloride, bensultap, thiosultap, monosultap (another name thiosultap-monosodium), bisultap (another name thiosultap-disodium) and polythialan;
organic chlorine compounds such as dicofol, tetradifon, endosulfan, dienochlor, dieldrin and methoxychlor;
organic metal compounds such as fenbutatin oxide and cyhexatin;
pyrethroid compounds such as fenvalerate, permethrin, cypermethrin, alpha-cypermethrin, zeta-cypermethrin, theta-cypermethrin, beta-cypermethrin, deltamethrin, cyhalothrin, gamma-cyhalothrin, lambda-cyhalothrin, tefluthrin, kappa-tefluthrin, ethofenprox, flufenprox, cyfluthrin, beta-cyfluthrin, fenpropathrin, flucythrinate, fluvalinate, cycloprothrin, pyrethrins, esfenvalerate, tetramethrin, resmethrin, protrifenbute, bifenthrin, kappa-bifenthrin, acrinathrin, allethrin, tau-fluvalinate, tralomethrin, profluthrin, metofluthrin, epsilon-metofluthrin, heptafluthrin, phenothrin, flumethrin, momfluorothrin, epsilon-momfluorothrin, silafluofen and chloroprallethrin;
benzoylurea compounds such as diflubenzuron, chlorfluazuron, teflubenzuron, flufenoxuron, lufenuron, novaluron, triflumuron, hexaflumuron, bistrifluron, noviflumuron, fluazuron and flufenoxuron;
juvenile hormone-like compounds such as methoprene, pyriproxyfen, fenoxycarb and diofenolan;
pyridazinone compounds such as pyridaben;
pyrazole compounds such as fenpyroximate, fipronil, ethiprole, acetoprole, pyrafluprole, pyriprole, cyenopyrafen and flufiprole;
pyrazole carboxamide compounds such as pyflubumide, tebufenpyrad, tolfenpyrad and dimpropyridaz;
pyridylpyrazole compounds such as chlorantraniliprole, cyantraniliprole, cyclaniliprole, tetraniliprole, tyclopyrazoflor, fluchlordiniliprole and tiorantraniliprole;
neonicotinoid compounds such as imidacloprid, nitenpyram, acetamiprid, thiacloprid, thiamethoxam, clothianidin, nidinotefuran, dinotefuran and nithiazine;
hydrazine compounds such as tebufenozide, methoxyfenozide, chromafenozide and halofenozide;
pyridine compounds such as pyridalyl, flonicamid and flumetnicam;
tetronic acid compounds such as spirodiclofen, spiromesifen and spirobudifen;
tetramic acid compounds such as spirotetramat and spiropidion;
strobilurin compounds such as fluacrypyrim, bifemetstrobin, pyriminostrobin and flupyroxystrobin;
pyrimidinamine compounds such as flufenerim and pyrimidifen;
organic sulfur compounds such as malathion;
triazine compounds such as cyromazine;
hydrazone compounds such as hydramethylnon;
diamide compounds such as flubendiamide, broflanilide, cyhalodiamide, pioxaniliprole and piperflanilide;
thiourea compounds such as diafenthiuron and chloromethiuron;
formamidine compounds such as amitraz, chlordimeform and chloromebuform;
pyridine azomethine compounds such as pymetrozine and pyrifluquinazone;
isoxazolinone compounds such as afoxolaner, fluralaner, fluxametamide, sarolaner and isoflualanam;
other compounds such as buprofezin, hexythiazox, triazamate, chlorfenapyr, indoxacarb, acequinocyl, etoxazole, 1,3-dichloropropene, benclothiaz, bifenazate, propargite, clofentezine, metaflumizone, cyflumetofen, fenazaquin, amidoflumet, sulfluramid, hydramethylnon, metaldehyde, sulfoxaflor, fluensulfone, verbutin, dicloromezotiaz, triflumezopyrim, fluhexafon, tioxazafen, afidopyropen, flometoquin, flupyradifurone, fluazaindolizine, acynonapyr, benzpyrimoxan, flupyrimin, oxazosulfyl, sulfiflumin, bisulflufen, cybenzoxasulfyl, galquin, tiapyrachlor and bentioflumin.

Further, the present composition may be mixed with or used in combination with the following compounds.

Microbial agricultural chemicals, such as insecticidal crystal proteins produced by Bacillus thuringiensis such as Bacillus thuringiensis aizawai, Bacillus thuringiensis kurstaki, Bacillus thuringiensis israelensis, Bacillus thuringiensis japonensis, Bacillus thuringiensis tenebrionis, insect viruses, entomopathogenic fungi, and nematophagous fungi:
antibiotics or semisynthetic antibiotics, such as abermectin, emamectin benzoate, ivermectin, milbemectin, milbemycin oxime, lepimectin, spinosad and spinetoram;
natural products, such as azadirachtin, rotenone, and ryanodine;
repellents, such as deet;
physical preservatives such as a paraffin oil and a mineral oil; and
RNAi pesticides such as ledprona and vadescana.

Preferred embodiments of the present invention will be described below. It should be understood that the present invention is by no means restricted thereto.
[1] A N-substituted oxy-2-aminothiazolecarboxamide compound represented by the formula (I) or a salt thereof.
[2] The N-substituted oxy-2-aminothiazolecarboxamide compound or a salt thereof according to [1], wherein R¹ is a (C₁-C₆) alkyl, (C₂-C₆) alkynyl or (C₂-C₆) alkenyl, which may be substituted with at least one T¹.
[3] The N-substituted oxy-2-aminothiazolecarboxamide compound or a salt thereof according to [1], wherein R¹ is a (C₁-C₆) alkyl or (C₂-C₆) alkynyl, which may be substituted with at least one T¹.
[4] The N-substituted oxy-2-aminothiazolecarboxamide compound or a salt thereof according to [1], wherein R¹ is a (C₁-C₆) alkyl, a (C₂-C₆) alkynyl or a (C₂-C₆) alkenyl.
[5] The N-substituted oxy-2-aminothiazolecarboxamide compound or a salt thereof according to[1], wherein R¹ is a (C₁-C₆) alkyl or a (C₂-C₆) alkynyl.
[6] The N-substituted oxy-2-aminothiazolecarboxamide compound or a salt thereof according to [1], wherein R¹ is a (C₁-C₃) alkyl or a (C₂-C₃) alkynyl.
[7] The N-substituted oxy-2-aminothiazolecarboxamide compound or a salt thereof according to [1], wherein R¹ is methyl, ethyl, propargyl, cyanomethyl, methoxycarbonylmethyl or ally.
[8] The N-substituted oxy-2-aminothiazolecarboxamide compound or a salt thereof according to [1], wherein R¹ is methyl, ethyl, propargyl or allyl.
[9] The N-substituted oxy-2-aminothiazolecarboxamide compound or a salt thereof according to [1], wherein R¹ is methyl, ethyl or propargyl.
[10] The N-substituted oxy-2-aminothiazolecarboxamide compound or a salt thereof according to [1], wherein R¹ is methyl or propargyl.
[11] The N-substituted oxy-2-aminothiazolecarboxamide compound or a salt thereof according to [1], wherein R¹ is methyl or ethyl.
[12] The N-substituted oxy-2-aminothiazolecarboxamide compound or a salt thereof according to [1], wherein R¹ is methyl.
[13] The N-substituted oxy-2-aminothiazolecarboxamide compound or a salt thereof according to [1], wherein R¹ is ethyl.
[14] The N-substituted oxy-2-aminothiazolecarboxamide compound or a salt thereof according to [1], wherein R¹ is propargyl.
[15] The N-substituted oxy-2-aminothiazolecarboxamide compound or a salt thereof according to any one of [1] to [14], wherein Y¹ is a halogen, a (C₁-C₆) alkyl, a (C₂-C₆) alkenyl, a (C₁-C₆) haloalkyl, cyano or nitro.
[16] The N-substituted oxy-2-aminothiazolecarboxamide compound or a salt thereof according to any one of [1] to [14], wherein Y¹ is a halogen, a (C₁-C₃) alkyl, a (C₂-C₃)alkenyl, a (C₁-C₃)haloalkyl, cyano or nitro.
[17] The N-substituted oxy-2-aminothiazolecarboxamide compound or a salt thereof according to any one of [1] to [14], wherein Y¹ is a fluorine atom, a chlorine atom, a hydrogen atom, methyl, trifluoromethyl, vinyl, cyano or nitro.
[18] The N-substituted oxy-2-aminothiazolecarboxamide compound or a salt thereof according to any one of [1] to [14], wherein Y¹ and Y⁴ are not hydrogen atoms at the same time.
[19] The N-substituted oxy-2-aminothiazolecarboxamide compound or a salt thereof according to any one of [1] to [14], wherein Y² and Y³ are each independently a halogen, a (C₁-C₆) alkyl or a hydrogen atom.
[20] The N-substituted oxy-2-aminothiazolecarboxamide compound or a salt thereof according to any one of [1] to [14], wherein Y² and Y³ are each independently a halogen, a (C₁-C₃) alkyl or a hydrogen atom.
[21] The N-substituted oxy-2-aminothiazolecarboxamide compound or a salt thereof according to any one of [1] to [14], wherein Y² and Y³ are each independently a fluorine atom, a chlorine atom, methyl or a hydrogen atom.
[22] The N-substituted oxy-2-aminothiazolecarboxamide compound or a salt thereof according to any one of [1] to [14], wherein Y⁴ is a halogen or a hydrogen atom.
[23] The N-substituted oxy-2-aminothiazolecarboxamide compound or a salt thereof according to any one of [1] to [14], wherein Y⁴ is a chlorine atom or a hydrogen atom.
[24] The N-substituted oxy-2-aminothiazolecarboxamide compound or a salt thereof according to any one of [1] to [14], wherein Y¹, Y², Y³ and Y⁴ are each independently a halogen, a hydrogen atom, a (C₁-C₃) alkyl, a (C₂-C₃) alkenyl, a (C₁-C₃) haloalkyl, cyano or nitro.
[25] The N-substituted oxy-2-aminothiazolecarboxamide compound or a salt thereof according to any one of [1] to [14], wherein Y¹, Y², Y³ and Y⁴ are each independently a halogen, a hydrogen atom, a (C₁-C₆) alkyl, a (C₁-C₆) haloalkyl or nitro.
[26] The N-substituted oxy-2-aminothiazolecarboxamide compound or a salt thereof according to any one of [1] to [14], wherein Y¹, Y², Y³ and Y⁴ are each independently a halogen, a hydrogen atom, a (C₁-C₃) alkyl, a (C₁-C₃)haloalkyl or nitro.
[27] The N-substituted oxy-2-aminothiazolecarboxamide compound or a salt thereof according to any one of [1] to [14], wherein Y¹, Y², Y³ and Y⁴ are each independently a halogen, a hydrogen atom, methyl, trifluoromethyl, vinyl, cyano or nitro.
[28] The N-substituted oxy-2-aminothiazolecarboxamide compound or a salt thereof according to any one of [1] to [14], wherein Y¹, Y², Y³ and Y⁴ are each independently a fluorine atom, a chlorine atom, a hydrogen atom, methyl, trifluoromethyl, vinyl, cyano or nitro.
[29] The N-substituted oxy-2-aminothiazolecarboxamide compound or a salt thereof according to any one of [1] to [14], wherein Y¹ and Y⁴ are each independently a hydrogen atom, a halogen, a (C₁-C₆) alkyl, a (C₁-C₆) haloalkyl, a (C₂-C₆) alkenyl, cyano or nitro, provided that Y¹ and Y⁴ are not hydrogen atoms at the same time, and Y² and Y³ are each independently a halogen, a (C₁-C₆) alkyl or a hydrogen atom.
[30] The N-substituted oxy-2-aminothiazolecarboxamide compound or a salt thereof according to any one of [1] to [14], wherein Y¹ and Y⁴ are each independently a halogen, a hydrogen atom, a (C₁-C₆) alkyl, a (C₁-C₆) haloalkyl, cyano or nitro, provided that Y¹ and Y⁴ are not hydrogen atoms at the same time, and Y² and Y³ are each independently a halogen or a hydrogen atom.
[31] The N-substituted oxy-2-aminothiazolecarboxamide compound or a salt thereof according to any one of [1] to [14], wherein Y¹ and Y⁴ are each independently a hydrogen atom, a halogen, a (C₁-C₃) alkyl, a (C₁-C₃) haloalkyl, a (C₂-C₃) alkenyl, cyano or nitro, provided that Y¹ and Y⁴ are not hydrogen atoms at the same time, and Y² and Y³ are each independently a fluorine atom, a chlorine atom, methyl or a hydrogen atom.
[32] The N-substituted oxy-2-aminothiazolecarboxamide compound or a salt thereof according to any one of [1] to [14], wherein Y¹ and Y⁴ are each independently a hydrogen atom, a fluorine atom, a chlorine atom, methyl, vinyl, trifluoromethyl, cyano or nitro, provided that Y¹ and Y⁴ are not hydrogen atoms at the same time, and Y² and Y³ are each independently a fluorine atom, a chlorine atom or a hydrogen atom.
[33] The N-substituted oxy-2-aminothiazolecarboxamide compound or a salt thereof according to any one of [1] to [14], wherein Y¹ is a halogen, a (C₁-C₆) alkyl, a (C₁-C₆) haloalkyl, a (C₂-C₆) alkenyl, cyano or nitro, and Y², Y³ and Y⁴ are each independently a halogen, a (C₁-C₆) alkyl or a hydrogen atom.
[34] The N-substituted oxy-2-aminothiazolecarboxamide compound or a salt thereof according to any one of [1] to [14], wherein Y⁴ is a halogen, a (C₁-C₆) alkyl, a (C₁-C₆) haloalkyl, a (C₂-C₆) alkenyl, cyano or nitro, and Y¹, Y² and Y³ are each independently a halogen, a (C₁-C₆) alkyl or a hydrogen atom.
[35] The N-substituted oxy-2-aminothiazolecarboxamide compound or a salt thereof according to any one of [1] to [14], wherein Y¹ is a halogen, a (C₁-C₆) alkyl, a (C₁-C₆) haloalkyl or nitro, and Y², Y³ and Y⁴ are each independently a halogen or a hydrogen atom.
[36] The N-substituted oxy-2-aminothiazolecarboxamide compound or a salt thereof according to any one of [1] to [14], wherein Y⁴ is a halogen, a (C₁-C₆) alkyl, a (C₁-C₆) haloalkyl or nitro, and Y¹, Y² and Y³ are each independently a halogen or a hydrogen atom.
[37] The N-substituted oxy-2-aminothiazolecarboxamide compound or a salt thereof according to any one of [1] to [14], wherein Y¹ is a halogen, a (C₁-C₃) alkyl, a (C₁-C₃) haloalkyl or nitro, and Y², Y³ and Y⁴ are each independently a fluorine atom, a chlorine atom or a hydrogen atom.
[38] The N-substituted oxy-2-aminothiazolecarboxamide compound or a salt thereof according to any one of [1] to [14], wherein Y⁴ is a halogen, a (C₁-C₃) alkyl, a (C₁-C₃) haloalkyl or nitro, and Y¹, Y² and Y³ are each independently a fluorine atom, a chlorine atom or a hydrogen atom.
[39] The N-substituted oxy-2-aminothiazolecarboxamide compound or a salt thereof according to any one of [1] to [14], wherein Y¹ is a fluorine atom, a chlorine atom, methyl, trifluoromethyl, cyano or nitro, and Y², Y³ and Y⁴ are each independently a fluorine atom, a chlorine atom or a hydrogen atom.
[40] The N-substituted oxy-2-aminothiazolecarboxamide compound or a salt thereof according to any one of [1] to [14], wherein Y⁴ is a fluorine atom, a chlorine atom, methyl, trifluoromethyl, cyano or nitro, and Y¹, Y² and Y³ are each independently a fluorine atom, a chlorine atom or a hydrogen atom.
[41] The N-substituted oxy-2-aminothiazolecarboxamide compound or a salt thereof according to any one of [1] to [14], wherein Y¹ is a fluorine atom, a chlorine atom, methyl, trifluoromethyl or nitro, Y² and Y³ are each independently a fluorine atom or a hydrogen atom, and Y⁴ is a hydrogen atom.
[42] The N-substituted oxy-2-aminothiazolecarboxamide compound or a salt thereof according to any one of [1] to [14], wherein Y¹ is a fluorine atom, a chlorine atom or methyl, Y² and Y³ are each independently a fluorine atom or a hydrogen atom, and Y⁴ is a hydrogen atom.
[43] The N-substituted oxy-2-aminothiazolecarboxamide compound or a salt thereof according to any one of [1] to [14], wherein Y¹ is a fluorine atom, a chlorine atom or trifluoromethyl, Y² and Y³ are each independently a fluorine atom or a hydrogen atom, and Y⁴ is a hydrogen atom.
[44] The N-substituted oxy-2-aminothiazolecarboxamide compound or a salt thereof according to any one of [1] to [14], wherein Y¹ is a fluorine atom, a chlorine atom or nitro, Y² and Y³ are each independently a fluorine atom or a hydrogen atom, and Y⁴ is a hydrogen atom.
[45] The N-substituted oxy-2-aminothiazolecarboxamide compound or a salt thereof according to any one of [1] to [14], wherein Y¹ is a fluorine atom, a chlorine atom or cyano, Y² and Y³ are each independently a fluorine atom or a hydrogen atom, and Y⁴ is a hydrogen atom.
[46] The N-substituted oxy-2-aminothiazolecarboxamide compound or a salt thereof according to any one of [1] to [14], wherein Y¹ is a fluorine atom or a chlorine atom, Y² and Y³ are each independently a fluorine atom or a hydrogen atom, and Y⁴ is a hydrogen atom.
[47] The N-substituted oxy-2-aminothiazolecarboxamide compound or a salt thereof according to any one of [1] to [14], wherein Y¹ is methyl, Y² and Y³ are each independently a fluorine atom or a hydrogen atom, and Y⁴ is a hydrogen atom.
[48] The N-substituted oxy-2-aminothiazolecarboxamide compound or a salt thereof according to any one of [1] to [14], wherein Y¹ is trifluoromethyl, Y² and Y³ are each independently a fluorine atom or a hydrogen atom, and Y⁴ is a hydrogen atom.
[49] The N-substituted oxy-2-aminothiazolecarboxamide compound or a salt thereof according to any one of [1] to [14], wherein Y¹ is nitro, Y² and Y³ are each independently a fluorine atom or a hydrogen atom, and Y⁴ is a hydrogen atom.
[50] The N-substituted oxy-2-aminothiazolecarboxamide compound or a salt thereof according to any one of [1] to [14], wherein Y¹ is cyano, Y² and Y³ are each independently a fluorine atom or a hydrogen atom, and Y⁴ is a hydrogen atom.
[51] The N-substituted oxy-2-aminothiazolecarboxamide compound or a salt thereof according to any one of [1] to [14], wherein Y⁴ is a fluorine atom or a chlorine atom, Y² and Y³ are each independently a fluorine atom or a hydrogen atom, and Y¹ is a hydrogen atom.
[52] The N-substituted oxy-2-aminothiazolecarboxamide compound or a salt thereof according to any one of [1] to [14], wherein Y⁴ is a chlorine atom, Y² and Y³ are each independently a fluorine atom or a hydrogen atom, and Y¹ is a hydrogen atom.
[53] The N-substituted oxy-2-aminothiazolecarboxamide compound or a salt thereof according to any one of [1] to [52], wherein X¹ is a halogen, a (C₁-C₃) alkyl, a (C₁-C₃) haloalkyl, cyano or a hydrogen atom.
[54] The N-substituted oxy-2-aminothiazolecarboxamide compound or a salt thereof according to any one of [1] to [52], wherein X¹ is a halogen, a (C₁-C₆) alkyl, a (C₁-C₆) haloalkyl or a hydrogen atom.
[55] The N-substituted oxy-2-aminothiazolecarboxamide compound or a salt thereof according to any one of [1] to [52], wherein X¹ is a halogen, a (C₁-C₃) alkyl, a (C₁-C₃) haloalkyl or a hydrogen atom.
[56] The N-substituted oxy-2-aminothiazolecarboxamide compound or a salt thereof according to any one of [1] to [52], wherein X¹ is a halogen.
[57] The N-substituted oxy-2-aminothiazolecarboxamide compound or a salt thereof according to any one of [1] to [52], wherein X¹ is a (C₁-C₃) alkyl.
[58] The N-substituted oxy-2-aminothiazolecarboxamide compound or a salt thereof according to any one of [1] to [52], wherein X¹ is a (C₁-C₃) haloalkyl.
[59] The N-substituted oxy-2-aminothiazolecarboxamide compound or a salt thereof according to any one of [1] to [52], wherein X¹ is a fluorine atom, a chlorine atom, methyl, difluoromethyl, trifluoromethyl, cyano or a hydrogen atom.
[60] The N-substituted oxy-2-aminothiazolecarboxamide compound or a salt thereof according to any one of [1] to [52], wherein X¹ is a fluorine atom, a chlorine atom, difluoromethyl, trifluoromethyl or a hydrogen atom.
[61] The N-substituted oxy-2-aminothiazolecarboxamide compound or a salt thereof according to any one of [1] to [52], wherein X¹ is a fluorine atom, a chlorine atom, methyl or a hydrogen atom.
[62] The N-substituted oxy-2-aminothiazolecarboxamide compound or a salt thereof according to any one of [1] to [52], wherein X¹ is a fluorine atom, a chlorine atom or methyl.
[63] The N-substituted oxy-2-aminothiazolecarboxamide compound or a salt thereof according to any one of [1] to [52], wherein X¹ is a fluorine atom or a chlorine atom.
[64] The N-substituted oxy-2-aminothiazolecarboxamide compound or a salt thereof according to any one of [1] to [52], wherein X¹ is difluoromethyl or trifluoromethyl.
[65] The N-substituted oxy-2-aminothiazolecarboxamide compound or a salt thereof according to any one of [1] to [52], wherein X¹ is a fluorine atom.
[66] The N-substituted oxy-2-aminothiazolecarboxamide compound or a salt thereof according to any one of [1] to [52], wherein X¹ is a chlorine atom.
[67] The N-substituted oxy-2-aminothiazolecarboxamide compound or a salt thereof according to any one of [1] to [52], wherein X¹ is methyl.
[68] The N-substituted oxy-2-aminothiazolecarboxamide compound or a salt thereof according to any one of [1] to [52], wherein X¹ is a hydrogen atom.
[69] The N-substituted oxy-2-aminothiazolecarboxamide compound or a salt thereof according to [1], wherein R¹ is a (C₁-C₆) chain hydrocarbon, X¹ is a halogen, a (C₁-C₆) alkyl, a (C₁-C₆) haloalkyl or a hydrogen atom, and Y¹, Y², Y³ and Y⁴ are each independently a halogen, a hydrogen atom, a (C₁-C₆) alkyl, a (C₁-C₆) haloalkyl or nitro.
[70] The N-substituted oxy-2-aminothiazolecarboxamide compound or a salt thereof according to [69], wherein R¹ is a (C₁-C₆) alkyl or a (C₂-C₆) alkynyl.
[71] The N-substituted oxy-2-aminothiazolecarboxamide compound or a salt thereof according to [69], wherein R¹ is a (C₁-C₃) alkyl or a (C₂-C₃) alkynyl.
[72] The N-substituted oxy-2-aminothiazolecarboxamide compound or a salt thereof according to [69], wherein R¹ is methyl or propargyl.
[73] The N-substituted oxy-2-aminothiazolecarboxamide compound or a salt thereof according to any one of [69] to [72], wherein Y¹ is a halogen, a (C₁-C₆) alkyl, a (C₁-C₆) haloalkyl or nitro, and Y², Y³ and Y⁴ are each independently a halogen or a hydrogen atom.
[74] The N-substituted oxy-2-aminothiazolecarboxamide compound or a salt thereof according to any one of [69] to [72], wherein Y¹ is a halogen, a (C₁-C₃) alkyl, a (C₁-C₃) haloalkyl or nitro, and Y², Y³ and Y⁴ are each independently a fluorine atom, a chlorine atom or a hydrogen atom.
[75] The N-substituted oxy-2-aminothiazolecarboxamide compound or a salt thereof according to any one of [69] to [72], wherein Y¹ is a fluorine atom, a chlorine atom, methyl, trifluoromethyl or nitro, Y² and Y³ are each independently a fluorine atom or a hydrogen atom, and Y⁴ is a hydrogen atom.
[76] The N-substituted oxy-2-aminothiazolecarboxamide compound or a salt thereof according to any one of [69] to [75], wherein X¹ is a halogen, a (C₁-C₃) alkyl, a (C₁-C₃) haloalkyl or a hydrogen atom.
[77] The N-substituted oxy-2-aminothiazolecarboxamide compound or a salt thereof according to any one of [69] to [75], wherein X¹ is a fluorine atom, a chlorine atom, difluoromethyl, trifluoromethyl or a hydrogen atom.
[78] An agricultural and horticultural fungicide, which comprises as an active ingredient the N-substituted oxy-2-aminothiazolecarboxamide compound or a salt thereof as defined in any one of [1] to [77].
[79] A method for controlling harmful plant diseases, which comprises applying an effective amount of the N-substituted oxy-2-aminothiazolecarboxamide compound or a salt thereof as defined in any one of [1] to [77] to a plant body, phytopathogen or soil.

### [EXAMPLE 1]

Now, examples of the present invention will be described, however, the present invention is by no means restricted thereto. The melting point as a physical property of the Invention Compound was measured by a melting point measuring apparatus (model M-565, manufactured by Buchi). The ¹H-NMR spectrum data was measured in a measurement solvent by a FT-NMR apparatus (product name: JNM-ECX (500 MHz) manufactured by JEOL Ltd. or AVANCE III HD (300 MHz) manufactured by Bruker) (¹H nuclear magnetic resonance spectroscopy). The measurement solvent includes tetramethylsilane (TMS) as an internal standard in some cases.

In this specification, room temperature means about 10 to 30°C.

### [Synthesis Example]

### Synthesis Example 1: Synthesis of 2-amino-4-chloro-N-{2-chloro-5,6-difluoropyridin- 3-yl)methyl}-N-methoxythiazole-5-carboxamide (compound No. 17)

### (1) (2-chloro-5,6-difluoropyridin-3-yl)methanol:

To a solution of 2-chloro-3-cyano-5,6-difluoropyridine (2.0 g) described in Example 2, Step 1 in WO2016/097862, at page 67, in dichloromethane (60 mL), a solution of diisobutylaluminum hydride in hexane (1.03M, 36.9 mL) was added dropwise under a nitrogen atmosphere at -78°C, and the obtained mixture was stirred at the same temperature for 1.5 hours. Methanol (1.8 mL) and 10% aqueous hydrochloric acid solution (18 mL) were sequentially added, followed by stirring at the same temperature for 30 minutes and by stirring at room temperature for 10 minutes. The organic layer was separated, and the aqueous layer was neutralized with a saturated aqueous sodium hydrogen carbonate solution and subjected to filtration with Celite. The filtrate was extracted with ethyl acetate. The organic layer obtained by extraction was sequentially washed with saturated aqueous potassium sodium tartrate solution, water and brine, dried over anhydrous sodium sulfate, subjected to filtration and concentrated under reduced pressure to obtain oily crude product of 2-chloro-5,6-difluoronicotinaldehyde (2.03 g).

The crude product (2.03 g) was dissolved in methanol (100 mL), sodium borohydride (0.52 g) was added to the obtained solution at 0°C, and the obtained mixture was stirred at room temperature overnight to obtain a reaction solution. The reaction solution was quenched with an aqueous hydrochloric acid solution. The quenched reaction solution was neutralized with saturated aqueous sodium hydrogen carbonate solution. Methanol was removed by distillation, and ethyl acetate was added to the obtained residue. The organic layer was separated, and the aqueous layer was extracted with ethyl acetate. The organic layer obtained by extraction was sequentially washed with water and brine, dried over anhydrous sodium sulfate, subjected to filtration and concentrated under reduced pressure. The obtained residue was purified by column chromatography (eluent: ethyl acetate/heptane) to obtain oily (2-chloro-5,6-difluoropyridin-3-yl)methanol (1.49 g). Its ¹H-NMR spectrum data was as follows.
¹H NMR (CDCl₃/300 MHz):δ(ppm)= 7.85 (t, 1H), 4.76 (d, 2H), 2.07 (t, 1H).

### (2) N-[(2-chloro-5,6-difluoropyridin-3-yl)methyl]-O-methylhydroxylamine:

(i)
   (2-chloro-5,6-difluoropyridin-3-yl)methanol (294 mg) and triethylamine (0.46 mL) were mixed with tetrahydrofuran (15 mL). To the obtained mixture, methanesulfonyl chloride (0.14 mL) was added at 0°C, followed by stirring at room temperature for 4.5 hours to obtain a reaction solution. The reaction solution was quenched with saturated aqueous sodium hydrogen carbonate solution. To the quenched reaction solution, ethyl acetate was added, and the aqueous layer was extracted with ethyl acetate. The organic layer obtained by extraction was sequentially washed with water and brine, dried over anhydrous sodium sulfate, subjected to filtration and concentrated under reduced pressure to obtain oily crude product of (2-chloro-5,6-difluoropyridin-3-yl)methyl methanesulfonate (409 mg).
(ii)
   The crude product (409 mg) obtained in the above (2) (i) was dissolved in acetone (15 mL), and to the obtained solution, lithium bromide (207 mg) was added at room temperature, followed by stirring under reflux with heating for 2.5 hours. After cooling to room temperature, the reaction solution was quenched with water. To the quenched reaction solution, ethyl acetate was added, and the aqueous layer was extracted with ethyl acetate. The organic layer obtained by extraction was sequentially washed with water and brine, dried over anhydrous sodium sulfate, subjected to filtration and concentrated under reduced pressure to obtain oily crude product of 3-(bromomethyl)-2-chloro-5,6-difluoropyridine (233 mg).
(iii)
   The crude product (233 mg) obtained in the above (2) (ii), diisopropylethylamine (0.5 mL) and N,N-dimethylformamide (5 mL) were mixed to obtain a mixture. To the obtained mixture, O-methylhydroxylamine hydrochloride (96.31 mg) was added at room temperature, followed by stirring at 50°C for 7.5 hours to obtain a reaction solution. After the obtained reaction solution was cooled to room temperature, the reaction solution was quenched with water. To the quenched reaction solution, ethyl acetate and heptane were sequentially added, and the aqueous layer was extracted with ethyl acetate/heptane mixed solvent. The organic layer obtained by extraction was sequentially washed with water and brine, dried over anhydrous sodium sulfate, subjected to filtration and concentrated under reduced pressure. The obtained residue was purified by column chromatography (eluent: ethyl acetate/heptane) to obtain oily N-[(2-chloro-5,6-difluoropyridin-3-yl)methyl]-O-methyl hydroxylamine (102 mg). Its ¹H-NMR spectrum data was as follows.
¹H NMR (CDCl₃/300 MHz):δ(ppm)= 7.76 (t, 1H), 5.89 (brs, 1H), 4.11 (d, 2H), 3.53 (s, 3H).

### (3) 2-[(tert-butoxycarbonyl)amino]-4-chlorothiazole-5-carboxylic acid:

tert-butyl(4-chloro-5-formylthiazol-2-yl)carbamate (15.86 g) synthesized in accordance with Example 27, Step 1 in WO2008/063888 at page 108, and 2-methyl-2-butene (32 mL) were mixed with a mixed solvent of tetrahydrofuran (130 mL) and tert-butanol (130 mL) to obtain a solution. To the obtained solution, aqueous solution (22 mL) of sodium dihydrogen phosphate (14.49 g) was added at 0°C, followed by stirring to obtain a mixture. 15 minutes later, to the mixture, aqueous solution (44 mL) of sodium chlorite (13.6 g, 80%) was added, followed by stirring at room temperature overnight. Aqueous hydrochloric acid solution was added to the reaction solution to acidify the reaction solution, and ethyl acetate was added for extraction. The organic layer obtained by extraction was sequentially washed with water and brine, dried over anhydrous sodium sulfate, subjected to filtration and concentrated under reduced pressure. The obtained solid was dissolved in saturated aqueous sodium hydrogen carbonate solution, and the aqueous layer was washed with ethyl acetate. The aqueous layer washed with ethyl acetate was acidified with aqueous hydrochloric acid solution. To the acidified aqueous layer, ethyl acetate was added, and the aqueous layer was extracted with ethyl acetate. The organic layer obtained by extraction was sequentially washed with water and brine, dried over anhydrous sodium sulfate, subjected to filtration and concentrated under reduced pressure to obtain a solid. The obtained solid was washed with 50% ethyl acetate/heptane solution to obtain white solid 2-[(tert-butoxycarbonyl)amino]-4-chlorothiazole-5-carboxylic acid (15.57 g). Its ¹H-NMR spectrum data was as follows.
¹H NMR (DMSO-d₆/300 MHz):δ(ppm)= 12.19 (s, 1H), 1.49 (s, 9H).

### (4) tert-butyl[4-chloro-5-[{(2-chloro-5,6-difluoropyridin-3-yl)methyl}(methoxy)carbamoyl] thiazol-2-yl]carbamate:

To a solution of 2-[(tert-butoxycarbonyl)amino]-4-chlorothiazole-5-carboxylic acid (146.97 mg) in tetrahydrofuran (5 mL), under cooling with ice, oxalyl chloride (0.05 mL) and a catalytic amount of N,N-dimethylformamide were added, followed by stirring under a nitrogen atmosphere. 15 minutes later, the mixture was warmed to room temperature and stirred further for 30 minutes. The reaction solution was concentrated under reduced pressure to obtain 2-[(tert-butoxycarbonyl)amino]-4-chlorothiazole-5-carboxylic acid chloride (hereinafter sometimes referred to as acid chloride). Tetrahydrofuran (3 mL) was added to the obtained acid chloride to dissolve, and then a solution of N-[(2-chloro-5,6- difluoropyridin-3-yl)methyl]-O-methylhydroxylamine (100 mg) in tetrahydrofuran (2 mL) and diisopropylethylamine (0.13 mL) were sequentially added at room temperature, followed by stirring under a nitrogen atmosphere at 50°C for 2.5 hours to obtain a reaction solution. The obtained reaction solution was cooled to room temperature, and the reaction solution was quenched with saturated aqueous sodium hydrogen carbonate solution. To the quenched reaction solution, ethyl acetate was added, and the aqueous layer was extracted with ethyl acetate. The organic layer obtained by extraction was sequentially washed with water and brine, dried over anhydrous sodium sulfate, subjected to filtration and concentrated under reduced pressure. The obtained residue was purified by column chromatography (eluent: ethyl acetate/heptane) to obtain amorphous tert-butyl[4-chloro-5-[{(2-chloro-5,6-difluoropyridin-3-yl)methyl}(methoxy)carbamoyl]thiazol-2-yl]carbamate (189 mg). Its ¹H-NMR spectrum data was as follows.
¹H NMR (DMSO-d₆/300 MHz):δ(ppm)= 12.13 (brs, 1H), 8.12 (t, 1H), 5.01 (s, 2H), 3.72 (s, 3H), 1.49 (s, 9H).

### (5) 2-amino-4-chloro-N-{2-chloro-5,6-difluoropyridin-3-yl)methyl}-N-methoxythiazole-5-carboxamide (compound No. 17):

To a solution of tert-butyl[4-chloro-5-[{(2-chloro-5,6-difluoropyridin-3-yl)methyl} (methoxy)carbamoyl]thiazol-2-yl]carbamate (189 mg) in dichloromethane (3 mL), trifluoroacetic acid (0.62 mL) was added, followed by stirring overnight at 40°C to obtain a reaction solution. The obtained reaction solution was cooled to room temperature, and saturated aqueous sodium hydrogen carbonate solution was slowly added to the reaction solution to basify the reaction solution. The aqueous layer of the basified reaction solution was extracted with ethyl acetate. The organic layer obtained by extraction was sequentially washed with water and brine, dried over anhydrous sodium sulfate, subjected to filtration and concentrated under reduced pressure. The obtained residue was purified by column chromatography (eluent: ethyl acetate/heptane) to obtain the desired title compound (compound No. 17, 177 mg) as a solid. Its ¹H-NMR spectrum data was as follows.
¹H NMR (DMSO-d₆/300 MHz):δ(ppm)= 8.08-8.02 (m, 3H), 4.94 (s, 2H), 3.69 (s, 3H).

Now, representative examples of the compound (I) are specifically shown in Table 1. These compounds can be synthesized in accordance with the above Production Process and Synthesis Example, and methods known in this technical field.

In Table 1, No. represents Compound No. of the Invention Compound. In Table 1, Me is a methyl group, Et is an ethyl group, Pr is a n-propyl group, Hex is a n-hexyl group, NO₂ is a nitro group, - is a single bond, = is a double bond, and ≡ is a triple bond.

Further, in the case of salts of the compound (I), types of the salts are shown in the Remarks column in Table 1. For examples, a compound indicated with HCl salt in the Remarks column in Table 1, is a hydrochloride, a compound indicated with TsOH salt is a p-toluenesulfonate, and a compound indicated with Na salt is a sodium salt. For example, compound No. 68 is a hydrochloride of compound No. 17. Compound No. 69 is a p-toluenesulfonate of compound No. 17. Compound No. 70 is a sodium salt of compound No. 17.

**Table 1**

| 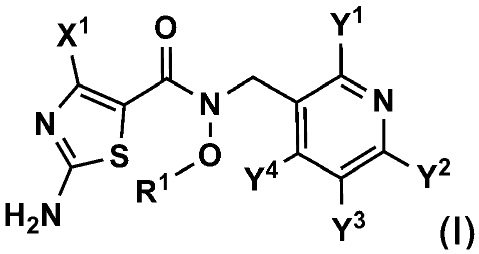 | | | | | | | |
|---|---|---|---|---|---|---|---|
| No. | X¹ | R¹ | Y¹ | Y² | Y³ | Y⁴ | Remarks |
| 1 | H | Me | Cl | H | H | H | |
| 2 | Cl | Me | Cl | H | H | H | |
| 3 | H | Me | H | H | H | Cl | |
| 4 | Cl | Me | H | H | H | Cl | |
| 5 | Cl | Me | Cl | F | H | H | |
| 6 | Br | Me | Cl | F | H | H | |
| 7 | F | Me | Cl | F | H | H | |
| 8 | Cl | Me | Cl | H | F | H | |
| 9 | Br | Me | Cl | H | F | H | |
| 10 | F | Me | Cl | H | F | H | |
| 11 | Cl | Me | H | F | H | Cl | |
| 12 | Br | Me | H | F | H | Cl | |
| 13 | F | Me | H | F | H | Cl | |
| 14 | Cl | Me | H | H | F | Cl | |
| 15 | Br | Me | H | H | F | Cl | |
| 16 | F | Me | H | H | F | Cl | |
| 17 | Cl | Me | Cl | F | F | H | |
| 18 | Br | Me | Cl | F | F | H | |
| 19 | F | Me | Cl | F | F | H | |
| 20 | Cl | Me | H | F | F | Cl | |
| 21 | Br | Me | H | F | F | Cl | |
| 22 | F | Me | H | F | F | Cl | |
| 23 | Cl | Me | F | F | H | H | |
| 24 | Br | Me | F | F | H | H | |
| 25 | F | Me | F | F | H | H | |
| 26 | Cl | Me | H | F | H | F | |
| 27 | Br | Me | H | F | H | F | |
| 28 | F | Me | H | F | H | F | |
| 29 | Cl | Me | F | H | F | H | |
| 30 | Br | Me | F | H | F | H | |
| 31 | F | Me | F | H | F | H | |
| 32 | Cl | Me | F | F | F | H | |
| 33 | Br | Me | F | F | F | H | |
| 34 | F | Me | F | F | F | H | |
| 35 | Cl | Me | Br | F | H | H | |
| 36 | Cl | Me | Br | H | F | H | |
| 37 | Cl | Me | H | H | H | Br | |
| 38 | Cl | Me | H | F | H | Br | |
| 39 | Cl | Me | Me | F | H | H | |
| 40 | Cl | Me | Me | H | F | H | |
| 41 | Cl | Me | Me | F | F | H | |
| 42 | Cl | Me | H | F | H | Me | |
| 43 | Cl | Me | NO₂ | H | H | H | |
| 44 | Cl | Me | NO₂ | F | H | H | |
| 45 | Cl | Me | NO₂ | H | F | H | |
| 46 | Cl | Me | H | F | H | NO₂ | |
| 47 | Cl | Me | CF₃ | H | H | H | |
| 48 | Cl | Me | H | H | H | CF₃ | |
| 49 | Cl | Me | CF₃ | F | H | H | |
| 50 | Cl | Me | H | F | H | CF₃ | |
| 51 | H | Me | Cl | F | F | H | |
| 52 | CHF₂ | Me | Cl | F | F | H | |
| 53 | CF₃ | Me | Cl | F | F | H | |
| 54 | Me | Me | Cl | F | F | H | |
| 55 | Cl | -CH₂C≡CH | Cl | F | F | H | |
| 56 | Br | -CH₂C≡CH | Cl | F | F | H | |
| 57 | F | -CH₂C≡CH | Cl | F | F | H | |
| 58 | Cl | Et | Cl | F | F | H | |
| 59 | Br | Et | Cl | F | F | H | |
| 60 | F | Et | Cl | F | F | H | |
| 61 | Cl | -CH₂CH=CH₂ | Cl | F | F | H | |
| 62 | Br | -CH₂CH=CH₂ | Cl | F | F | H | |
| 63 | F | -CH₂CH=CH₂ | Cl | F | F | H | |
| 64 | Cl | Me | CHF₂ | F | H | H | |
| 65 | Cl | Me | CHF₂ | H | F | H | |
| 66 | Cl | Me | CHF₂ | F | F | H | |
| 67 | Cl | Me | H | F | H | CHF₂ | |
| 68 | Cl | Me | Cl | F | F | H | HCl salt |
| 69 | Cl | Me | Cl | F | F | H | TsOH salt |
| 70 | Cl | Me | Cl | F | F | H | Na salt |
| 71 | Cl | Me | CN | H | H | H | |
| 72 | Cl | Me | CN | F | H | H | |
| 73 | Br | Me | CN | F | H | H | |
| 74 | F | Me | CN | F | H | H | |
| 75 | Cl | Me | CN | H | F | H | |
| 76 | Br | Me | CN | H | F | H | |
| 77 | F | Me | CN | H | F | H | |
| 78 | Cl | Me | CN | F | F | H | |
| 79 | Br | Me | CN | F | F | H | |
| 80 | F | Me | CN | F | F | H | |
| 81 | Cl | Me | H | F | H | CN | |
| 82 | Br | Me | H | F | H | CN | |
| 83 | F | Me | H | F | H | CN | |
| 84 | CN | Me | Cl | F | F | H | |
| 85 | Cl | Me | -CH=CH₂ | F | F | H | |
| 86 | Cl | Me | Et | F | F | H | |
| 87 | Cl | CH₂CN | Cl | F | F | H | |
| 88 | Br | CH₂CN | Cl | F | F | H | |
| 89 | F | CH₂CN | Cl | F | F | H | |
| 90 | Cl | CH₂COOMe | Cl | F | F | H | |
| 91 | Br | CH₂COOMe | Cl | F | F | H | |
| 92 | F | CH₂COOMe | Cl | F | F | H | |
| 93 | Cl | Me | Cl | Cl | F | H | |
| 94 | Cl | Me | Cl | Me | H | H | |
| 95 | Cl | Et | Cl | F | H | H | |
| 96 | Br | Et | Cl | F | H | H | |
| 97 | F | Et | Cl | F | H | H | |
| 98 | Cl | Et | Cl | H | F | H | |
| 99 | Br | Et | Cl | H | F | H | |
| 100 | F | Et | Cl | H | F | H | |
| 101 | Cl | Et | H | F | H | Cl | |
| 102 | Br | Et | H | F | H | Cl | |
| 103 | F | Et | H | F | H | Cl | |
| 104 | Cl | -CH₂C≡CH | Cl | F | H | H | |
| 105 | Br | -CH₂C≡CH | Cl | F | H | H | |
| 106 | F | -CH₂C≡CH | Cl | F | H | H | |
| 107 | Cl | -CH₂C≡CH | Cl | H | F | H | |
| 108 | Br | -CH₂C≡CH | Cl | H | F | H | |
| 109 | F | -CH₂C≡CH | Cl | H | F | H | |
| 110 | Cl | -CH₂C≡CH | H | F | H | Cl | |
| 111 | Br | -CH₂C≡CH | H | F | H | Cl | |
| 112 | F | -CH₂C≡CH | H | F | H | Cl | |
| 113 | Cl | -CH₂CH=CH₂ | Cl | F | H | H | |
| 114 | Br | -CH₂CH=CH₂ | Cl | F | H | H | |
| 115 | F | -CH₂CH=CH₂ | Cl | F | H | H | |
| 116 | Cl | -CH₂CH=CH₂ | Cl | H | F | H | |
| 117 | Br | -CH₂CH=CH₂ | Cl | H | F | H | |
| 118 | F | -CH₂CH=CH₂ | Cl | H | F | H | |
| 119 | Cl | -CH₂CH=CH₂ | H | F | H | Cl | |
| 120 | Br | -CH₂CH=CH₂ | H | F | H | Cl | |
| 121 | F | -CH₂CH=CH₂ | H | F | H | Cl | |
| 122 | Br | Me | Br | H | F | H | |
| 123 | F | Me | Br | H | F | H | |
| 124 | Cl | CH₂CN | Cl | F | H | H | |
| 125 | Br | CH₂CN | Cl | F | H | H | |
| 126 | F | CH₂CN | Cl | F | H | H | |
| 127 | Cl | CH₂CN | Cl | H | F | H | |
| 128 | Br | CH₂CN | Cl | H | F | H | |
| 129 | F | CH₂CN | Cl | H | F | H | |
| 130 | Cl | CH₂CN | H | F | H | Cl | |
| 131 | Br | CH₂CN | H | F | H | Cl | |
| 132 | F | CH₂CN | H | F | H | Cl | |
| 133 | Cl | CH₂COOMe | Cl | F | H | H | |
| 134 | Br | CH₂COOMe | Cl | F | H | H | |
| 135 | F | CH₂COOMe | Cl | F | H | H | |
| 136 | Cl | CH₂COOMe | Cl | H | F | H | |
| 137 | Br | CH₂COOMe | Cl | H | F | H | |
| 138 | F | CH₂COOMe | Cl | H | F | H | |
| 139 | Cl | CH₂COOMe | H | F | H | Cl | |
| 140 | Br | CH₂COOMe | H | F | H | Cl | |
| 141 | F | CH₂COOMe | H | F | H | Cl | |
| 142 | Cl | Me | H | H | H | F | |
| 143 | Br | Me | H | H | H | F | |
| 144 | F | Me | H | H | H | F | |
| 145 | Cl | Me | H | H | F | F | |
| 146 | Br | Me | H | H | F | F | |
| 147 | F | Me | H | H | F | F | |
| 148 | Cl | Me | H | F | F | F | |
| 149 | Br | Me | H | F | F | F | |
| 150 | F | Me | H | F | F | F | |
| 151 | Cl | Me | Br | F | F | H | |
| 152 | Br | Me | Br | F | F | H | |
| 153 | F | Me | Br | F | F | H | |
| 154 | Br | Me | H | F | H | Br | |
| 155 | F | Me | H | F | H | Br | |
| 156 | Cl | Me | F | H | H | H | |
| 157 | Br | Me | F | H | H | H | |
| 158 | F | Me | F | H | H | H | |
| 159 | Cl | Me | H | F | H | H | |
| 160 | Br | Me | H | F | H | H | |
| 161 | F | Me | H | F | H | H | |
| 162 | Cl | Me | H | H | F | H | |
| 163 | Br | Me | H | H | F | H | |
| 164 | F | Me | H | H | F | H | |
| 165 | Cl | Me | Me | H | H | H | |
| 166 | Br | Me | Me | H | H | H | |
| 167 | F | Me | Me | H | H | H | |
| 168 | Cl | Me | H | Cl | H | H | |
| 169 | Br | Me | H | Cl | H | H | |
| 170 | F | Me | H | Cl | H | H | |
| 171 | Cl | Me | Br | H | H | H | |
| 172 | Br | Me | Br | H | H | H | |
| 173 | F | Me | Br | H | H | H | |
| 174 | Cl | Me | CI | H | H | Cl | |
| 175 | Br | Me | CI | H | H | Cl | |
| 176 | F | Me | CI | H | H | Cl | |
| 177 | Cl | Me | CI | CF₃ | H | H | |
| 178 | Br | Me | CI | CF₃ | H | H | |
| 179 | F | Me | CI | CF₃ | H | H | |
| 180 | Cl | Me | F | H | H | F | |
| 181 | Br | Me | F | H | H | F | |
| 182 | F | Me | F | H | H | F | |
| 183 | Cl | Me | H | Cl | H | Cl | |
| 184 | Br | Me | H | Cl | H | Cl | |
| 185 | F | Me | H | Cl | H | Cl | |
| 186 | Cl | Me | CI | Cl | H | H | |
| 187 | Br | Me | CI | Cl | H | H | |
| 188 | F | Me | CI | Cl | H | H | |
| 189 | Cl | Me | Me | Me | H | H | |
| 190 | Br | Me | Me | Me | H | H | |
| 191 | F | Me | Me | Me | H | H | |
| 192 | Br | Me | H | H | H | Cl | |
| 193 | F | Me | H | H | H | Cl | |
| 194 | Cl | Me | H | H | H | Br | |
| 195 | Br | Me | H | H | H | Br | |
| 196 | F | Me | H | H | H | Br | |
| 197 | Cl | Me | H | H | H | Me | |
| 198 | Br | Me | H | H | H | Me | |
| 199 | F | Me | H | H | H | Me | |
| 200 | Cl | Me | Pr | F | F | H | |
| 201 | Cl | Me | Hex | CN | F | H | |
| 202 | Cl | Me | -(CH₂)₂CF₃ | NO₂ | F | H | |
| 203 | Cl | Me | -(CH₂)₅CF₃ | F | Cl | H | |
| 204 | Cl | Me | -CH=CH-CH₃ | F | Me | H | |
| 205 | Cl | Me | -CH=CH-(CH₂)₃CH₃ | F | CN | H | |
| 206 | Cl | Me | H | F | NO₂ | -CH=CH-CH₃ | |
| 207 | Cl | Me | H | F | CF₃ | -CH=CH-(CH₂)₃CH₃ | |
| 208 | Cl | Me | H | -CH=CH₂ | H | Pr | |
| 209 | Cl | Me | H | F | -CH=CH₂ | Hex | |
| 210 | Cl | Me | H | -(CH₂)₂CF₃ | H | -(CH₂)₂CF₃ | |
| 211 | Cl | Me | H | F | -(CH₂)₂CF₃ | -(CH₂)₅CF₃ | |
| 212 | Cl | Me | Cl | Pr | H | H | |
| 213 | Cl | Me | Cl | Hex | H | H | |
| 214 | Cl | Me | Cl | H | Pr | H | |
| 215 | Cl | Me | Cl | H | Hex | H | |

Physical properties of the compound (I) synthesized in accordance with the above Production Process and Example are shown in Tables 2 and 3. Table 2 illustrates the melting point of the compound (I). Table 3 illustrates ¹H-NMR spectrum data of the compound (I) (measured by ¹H-nuclear magnetic resonance spectroscopy; δ is the chemical shift value (ppm)). In Tables 2 and 3, No. is as defined for Table 1.

The melting point indicated with *1 in Table 2 represents a temperature at which the compound was decomposed when the melting point of the compound was to be measured.

In the ¹H-NMR spectrum data in Table 3, s is singlet, brs is broad singlet, d is doublet, t is triplet, q is quartet, and m is multiplet.

**Table 2**

| No. | Melting point (°C) |
|---|---|
| 3 | 276.1 |
| 7 | 207.8(*1) |
| 8 | 201.6 |
| 10 | 237.8(*1) |
| 11 | 210.0 |
| 13 | 223.4(*1) |
| 34 | 233.8(*1) |
| 41 | 206.5 |
| 43 | 198.8 |
| 47 | 227.0 |
| 51 | 213.7 |
| 53 | 149.3 |
| 54 | 153.2 |
| 55 | 170.3 |
| 57 | 201.1 |
| 75 | 200.7 |

**Table 3**

| No. | ¹H-NMR (300 MHz or 500 MHz, measurement solvent) δ (ppm) |
|---|---|
| 1 | (300 MHz, DMSO-d₆) 8.37 (dd, 1H), 7.79 (s, 1H), 7.74-7.71 (m, 3H), 7.45 (dd, 1H), 4.98 (s, 2H), 3.74 (s, 3H). |
| 2 | (500 MHz, acetone-d₆) 8.34-8.33 (m, 1H), 7.78-7.77 (m, 1H), 7.41 (dd, 1H), 7.20 (brs, 2H), 5.03 (s, 2H), 3.76 (s, 3H). |
| 17 | (300 MHz, DMSO-d₆) 8.08-8.02 (m, 3H), 4.94 (s, 2H), 3.69 (s, 3H). |
| 19 | (300 MHz, DMSO-d₆) 8.18 (brs, 2H), 8.02 (t, 1H), 4.91 (s, 2H), 3.70 (s, 3H). |
| 32 | (300 MHz, DMSO-d₆) 8.17 (m, 1H), 7.99 (brs, 2H), 4.89 (s, 2H), 3.70 (s, 3H). |
| 58 | (500 MHz, DMSO-d₆) 8.00 (t, 1H), 7.95 (brs, 2H), 4.90 (s, 2H), 3.92 (q, 2H), 1.15 (t, 3H). |
| 60 | (300 MHz, DMSO-d₆) 8.13 (brs, 2H), 8.00 (t, 1H), 4.90 (s, 2H), 3.97 (q, 2H), 1.20 (t, 3H). |
| 61 | (500 MHz, DMSO-d₆) 8.01 (t, 1H), 7.96 (brs, 2H), 5.91-5.82 (m, 1H), 5.36 (d, 1H), 5.28 (d, 1H), 4.91 (s, 2H), 4.42 (d, 2H). |
| 71 | (300 MHz, DMSO-d₆) 8.69 (dd, 1H), 8.01 (brs, 2H), 7.94 (dd, 1H), 7.74 (dd, 1H), 5.06 (s, 2H), 3.74 (s, 3H). |
| 84 | (500 MHz, DMSO-d₆) 8.13-8.07 (m, 3H), 4.99 (s, 2H), 3.73 (s, 3H). |
| 85 | (500 MHz, acetone-d₆) 7.82 (t, 1H), 7.20 (brs, 2H), 7.13 (dd, 1H), 6.29 (dd, 1H), 5.55, (dd, 1H), 5.07 (s, 2H), 3.72 (s, 3H). |
| 86 | (500 MHz, DMSO-d₆) 7.99 (brs, 2H), 7.81 (t, 1H), 4.93 (s, 2H), 3.66 (s, 3H), 2.73 (q, 2H), 1.15 (t, 3H). |
| 87 | (500 MHz, DMSO-d₆) 8.11 (brs, 2H), 8.00 (t, 1H), 5.02 (s, 2H), 4.97 (s, 2H). |
| 90 | (500 MHz, DMSO-d₆) 8.03-7.98 (m, 3H), 4.95 (s, 2H), 4.69 (s, 2H), 3.61 (s, 3H). |
| 93 | (500 MHz, DMSO-d₆) 7.98 (brs, 2H), 7.91 (d, 1H), 4.91 (s, 2H), 3.66 (s, 3H). |
| 94 | (500 MHz, DMSO-d₆) 7.95 (brs, 2H), 7.58 (d, 1H), 7.26 (d, 1H), 4.88 (s, 2H), 3.63 (s, 3H), 2.41 (s, 3H). |

Now, excellent controlling effects and usefulness of the compounds of the present invention against harmful plant diseases are explained with reference to specific Test Examples. As a control, each test was conducted using Example 68 described in Patent Document 1 (hereinafter sometimes referred to as Comparative Compound A), the structure of which is shown below.

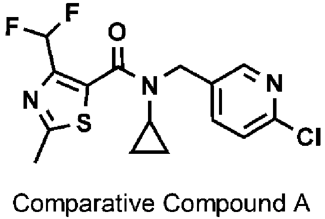

### [Test examples]

### Preparation of each chemical solution containing test compound:

The test compound and acetone or dimethyl sulfoxide were mixed to dissolve the test compound. The solution of the test compound was diluted with water to a predetermined concentration (400 ppm) of the test compound as an active ingredient to obtain chemical solutions. The chemical solutions thus obtained were used in the following Test Examples 1 to 3.

### Test Example 1: Test on fungicidal effect against tomato late blight (Phytophthora infestans) (test on preventive effect)

Tomato was cultivated in a polyethylene pot having a diameter of 6 cm, and when the tomato reached 4.5- to 5.5-leaf stage, 10 mL of the chemical solution was applied by means of a spray gun. After the chemical solution dried (the day of application), the tomato was sprayed and inoculated with a suspension of spores of Phytophthora infestans and placed in an inoculation box at a temperature of 20°C under a humidity of 95% or higher for 16 hours. Then, the tomato was placed in a constant temperature room at 20°C. 3 Days after the inoculation, the lesion area ratio was visually examined, and the control value was calculated in accordance with the following formula. As a result, chemical solutions containing each of the following Invention Compounds as the test compounds (active ingredient concentration: 400 ppm) had a control value of 80% or higher on tomato late blight. [Calculation of control value] $Control value \left(%\right) = 100 - \left(X/Y\right) \times 100$ X: lesion area ratio (%) in treated plot, Y: lesion area ratio (%) in non-treated plot
Test compounds: compound Nos. 1, 2, 3, 7, 8, 10, 11, 13, 17, 19, 32, 34, 41, 43, 47, 51, 53, 54, 55, 57, 58, 61, 71, 75, 84, 85, 86, 87, 90, 93, 94

On the other hand, a chemical solution containing Comparative Compound A (active ingredient concentration: 400 ppm) had a control value of less than 30%.

### Test Example 2: Test on fungicidal effect against cucumber downy mildew

### (Pseudoperonospora cubensis) (test on preventive effect)

Cucumber was cultivated in a polyethylene pot having a diameter of 6 cm, and when the cucumber reached 1.2- to 1.5-leaf stage, 10 mL of the chemical solution was applied by means of a spray gun. After the chemical solution dried (the day of application or the next day), the cucumber was sprayed and inoculated with a suspension of spores of Pseudoperonospora cubensis and placed in an inoculation box at a temperature of 20°C under a humidity of 95% or higher for 24 hours. Then, the cucumber was placed in a constant temperature room at 20°C. 7 Days after the inoculation, the lesion area ratio was examined on the same basis as in Text Example 1, and the control value was calculated in accordance with the same formula as in Test Example 1. As a result, chemical solutions containing each of the following Invention Compounds as test compounds (active ingredient concentration: 400 ppm) had a control value of 80% or higher on cucumber downy mildew.

Test Compounds: compound Nos. 1, 7, 8, 10, 11, 13, 19, 34, 41, 51, 53, 54, 55, 57, 58, 61, 75, 84, 85, 86, 87, 90, 93, 94

On the other hand, a chemical solution containing Comparative Compound A (active ingredient concentration: 400 ppm) had a control value of less than 30%.

### Test Example 3: Test on fungicidal effect against tomato late blight (Phytophthora infestans) (test on curative effect)

Tomato was cultivated in a polyethylene pot having a diameter of 6 cm, and when the tomato reached 4.5- to 5.5-leaf stage, the tomato was sprayed and inoculated with a suspension of spores of Phytophthora infestans and placed in an inoculation box at a temperature of 20°C under a humidity of 95% or higher for 4 hours. Then, 10 mL of the chemical solution was applied by means of a spray gun. After the chemical solution dried (the day of application), the tomato was placed in a constant temperature room at 20°C. 3 Days after the inoculation, the lesion area ratio was examined on the same basis as in Test Example 1, and the control value was calculated in accordance with the same formula as in Test Example 1. As a result, chemical solutions containing each of the following Invention Compounds as test compounds (active ingredient concentration: 400 ppm) had a control value of 80% or higher on tomato late blight.
Test Compounds: compound Nos. 3, 7, 8, 10, 11, 13, 17, 19, 32, 34, 41, 51, 54, 55, 57, 58, 61, 84, 87

On the other hand, a chemical solution containing Comparative Compound A (active ingredient concentration: 400 ppm) had a control value of less than 30%.

It was found from the above Test Examples that the Invention Compounds have excellent controlling effects against harmful plant diseases. Thus, the Invention Compounds are useful as an agricultural and horticultural fungicide.

Now, formulation of the Compound (I) will be specifically described. However, the mix ratio, the dosage form, etc. are not limited to the following Formulation Examples.

### FORMULATION EXAMPLE 1

| | |
|---|---|
| (1) Compound (I) | 20 parts by weight |
| (2) Clay | 72 parts by weight |
| (3) Sodium lignin sulfonate | 8 parts by weight |

The above components are uniformly mixed to obtain a wettable powder.

### FORMULATION EXAMPLE 2

| | |
|---|---|
| (1) Compound (I) | 5 parts by weight |
| (2) Talc | 95 parts by weight |

The above components are uniformly mixed to obtain a dust.

### FORMULATION EXAMPLE 3

| | |
|---|---|
| (1) Compound (I) | 20 parts by weight |
| (2) N,N-dimethylacetamide | 20 parts by weight |
| (3) Polyoxyethylene alkyl phenyl ether | 10 parts by weight |
| (4) Xylene | 50 parts by weight |

The above components are uniformly mixed and dissolved to obtain an emulsifiable concentrate.

### FORMULATION EXAMPLE 4

| | |
|---|---|
| (1) Clay | 68 parts by weight |
| (2) Sodium lignin sulfonate | 2 parts by weight |
| (3) Polyoxyethylene alkyl aryl sulfate | 5 parts by weight |
| (4) Pulverized silica | 25 parts by weight |

The mixture of the above components is mixed with the Compound (I) in a weight ratio of 4:1 to obtain a wettable powder.

### FORMULATION EXAMPLE 5

| | |
|---|---|
| (1) Compound (I) | 50 parts by weight |
| (2) Polyoxyethylene alkyl phenyl ether phosphate triethanolamine salt | 2 parts by weight |
| (3) Silicone | 0.2 part by weight |
| (4) Water | 47.8 parts by weight |

The above components are uniformly mixed and pulverized to obtain a base liquid, and

| | |
|---|---|
| (5) Sodium polycarboxylate | 5 parts by weight |
| (6) Anhydrous sodium sulfate | 42.8 parts by weight |

are added, and the mixture is uniformly mixed, granulated and dried to obtain a water-dispersible granule.

### FORMULATION EXAMPLE 6

| | |
|---|---|
| (1) Compound (I) | 5 parts by weight |
| (2) Polyoxyethylene octylphenyl ether | 1 part by weight |
| (3) Polyoxyethylene phosphoric acid ester | 0.1 part by weight |
| (4) Granular calcium carbonate | 93.9 parts by weight |

The above components (1) to (3) are preliminarily uniformly mixed and diluted with a proper amount of acetone, and then the mixture is sprayed onto the component (4), and acetone is removed to obtain a granule.

### FORMULATION EXAMPLE 7

| | |
|---|---|
| (1) Compound (I) | 2.5 parts by weight |
| (2) N-methyl-2-pyrrolidone | 2.5 parts by weight |
| (3) Soybean oil | 95.0 parts by weight |

The above components are uniformly mixed and dissolved to obtain an ultra low volume formulation.

### FORMULATION EXAMPLE 8

| | |
|---|---|
| (1) Compound (I) | 20 parts by weight |
| (2) Polyoxyethylene alkyl phenyl ether phosphate triethanolamine salt | 2 parts by weight |
| (3) Silicone | 0.2 part by weight |
| (4) Xanthan gum | 0.1 part by weight |
| (5) Ethylene glycol | 5 parts by weight |
| (6) Water | 72.7 parts by weight |

The above components are uniformly mixed and pulverized to obtain a water-based suspension.

The entire disclosure of Japanese Patent Application No. 2023-212115 filed on December 15, 2023 including specification, claims and abstract is incorporated herein by reference in its entirety.

## Claims

1. A N-substituted oxy-2-aminothiazolecarboxamide compound represented by the formula (I) or a salt thereof: 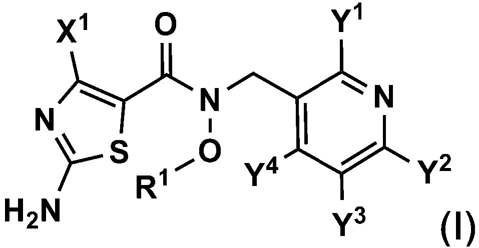
wherein R¹ is a (C₁-C₆) chain hydrocarbon which may be substituted with at least one T¹,
T¹ is cyano or -C(=O)O-R²,
R² is a (C₁-C₃) alkyl,
X¹ is a halogen, a (C₁-C₆) alkyl, a (C₁-C₆) haloalkyl, cyano or a hydrogen atom, and
Y¹, Y², Y³ and Y⁴ are each independently a halogen, a hydrogen atom, a (C₁-C₆) alkyl, a (C₂-C₆) alkenyl, a (C₁-C₆) haloalkyl, cyano or nitro.

2. The N-substituted oxy-2-aminothiazolecarboxamide compound or a salt thereof according to claim 1, wherein R¹ is a (C₁-C₆) alkyl or a (C₂-C₆) alkynyl.

3. The N-substituted oxy-2-aminothiazolecarboxamide compound or a salt thereof according to claim 1, wherein R¹ is methyl, ethyl or propargyl.

4. The N-substituted oxy-2-aminothiazolecarboxamide compound or a salt thereof according to any one of claims 1 to 3,
wherein Y¹ and Y⁴ are each independently a halogen, a hydrogen atom, a (C₁-C₆) alkyl, a (C₁-C₆) haloalkyl, cyano or nitro, provided that Y¹ and Y⁴ are not hydrogen atoms at the same time, and
Y² and Y³ are each independently a halogen or a hydrogen atom.

5. The N-substituted oxy-2-aminothiazolecarboxamide compound or a salt thereof according to claim 1, wherein
R¹ is a (C₁-C₆) chain hydrocarbon,
X¹ is a halogen, a (C₁-C₆) alkyl, a (C₁-C₆) haloalkyl or a hydrogen atom, and
Y¹, Y², Y³ and Y⁴ are each independently a halogen, a hydrogen atom, a (C₁-C₆) alkyl, a (C₁-C₆) haloalkyl or nitro.

6. The N-substituted oxy-2-aminothiazolecarboxamide compound or a salt thereof according to claim 5, wherein R¹ is a (C₁-C₆) alkyl or a (C₂-C₆) alkynyl.

7. The N-substituted oxy-2-aminothiazolecarboxamide compound or a salt thereof according to claim 5, wherein R¹ is methyl or propargyl.

8. The N-substituted oxy-2-aminothiazolecarboxamide compound or a salt thereof according to any one of claims 1 to 3 and 5,
wherein Y¹ is a halogen, a (C₁-C₆) alkyl, a (C₁-C₆) haloalkyl or nitro, and
Y², Y³ and Y⁴ are each independently a halogen or a hydrogen atom.

9. An agricultural and horticultural fungicide, which comprises as an active ingredient the N-substituted oxy-2-aminothiazolecarboxamide compound or a salt thereof as defined in any one of claims 1 to 3 and 5 to 7.

10. A method for controlling harmful plant diseases, which comprises applying an effective amount of the N-substituted oxy-2-aminothiazolecarboxamide compound or a salt thereof as defined in any one of claims 1 to 3 and 5 to 7 to a plant body, phytopathogen or soil.
